# EUROPEAN PATENT SPECIFICATION

(11) **EP 2 596 054 B1**
(45) Date of publication and mention of the grant of the patent: **02.03.2016**
(21) Application number: 11809349.1
(22) Date of filing: 16.06.2011
(51) Int. Cl.: C08J 5/18, C08L 3/10, C08K 5/053, C08L 67/02, C08L 101/16

(54) **BIODEGRADABLE FILMS**
BIOLOGISCH ABBAUBARE FILME
FILMS BIODÉGRADABLES

(30) Priority: 19.07.2010 US 839074
(43) Date of publication of application: 29.05.2013
(73) Proprietor: Kimberly-Clark Worldwide, Inc., Neenah, Wisconsin 54956 (US)
(72) Inventor: SHI, Bo, Neenah, Wisconsin 54956 (US); WANG, James, H., Appleton, Wisconsin 54911 (US)
(74) Representative: Beacham, Annabel Rose
(86) International application number: PCT/IB2011/052636
(87) International publication number: WO 2012/010991

(56) References cited:
- US-A- 5 217 803
- US-A- 6 054 510
- US-A1- 2002 006 989
- US-A1- 2008 147 034
- US-A1- 2009 054 548
- US-B1- 6 235 815

## Description

### Background of the Invention

Films are employed in a wide variety of disposable goods, such as diapers, sanitary napkins, adult incontinence garments, bandages, etc. For example, many sanitary napkins have an adhesive strip on the backside of the napkin (the napkin surface opposite to the body-contacting surface) to affix the napkin to an undergarment and hold the napkin in place against the body. Before use, the adhesive strip is protected with a peelable release liner. Once removed, the peelable release liner must be discarded. Other examples of films useful for disposable garments include baffle films for adult and feminine pads and liners, outercover films for diapers and training pants, and packaging films for the product bags of the disposable garment products. Such films, however, may not be biodegradable. Or, even if such films are biodegradable, they may include large quantities of expensive components that limit their usefulness in the disposable goods.

Biodegradable films that include biodegradable polyesters and thermoplastic starch have been made, but such films generally include more polyester than thermoplastic starch in order to avoid processing and/or performance problems that may occur. However, such films may be too expensive for use in disposable products, since biodegradable polyester is generally quite expensive. Another biodegradable film that includes a biodegradable polyester and a thermoplastic starch is disclosed in U.S. Patent Application Publication No. 2008/0147034.

As such, a need currently exists for a less expensive and biodegradable film that has mechanical properties suitable for use in disposable goods.

### Summary of the Invention

In accordance with one embodiment of the present invention, a biodegradable film is disclosed that includes from 1 wt.% to 49 wt. % by weight of the film of at least one biodegradable polyester, and from 46 wt.% to 75 wt.% by weight of the film of a thermoplastic oxidized starch comprising at least one oxidized corn starch or oxidized wheat starch and at least one plasticizer, further wherein the wt.% by weight of the film of the biodegradable polyester is less than the wt.% by weight of the film of the dispersed phase; wherein the biodegradability of the film and the polyester are determined according to ASTM Test Method 5338.92.

In accordance with another embodiment of the present invention, an absorbent article is disclosed that comprises the biodegradable film of the invention. The absorbent article preferably comprises a body portion that includes a liquid permeable topsheet, a generally liquid impermeable backsheet, and an absorbent core positioned between the backsheet and the topsheet. The absorbent article further comprises a release liner that defines a first surface and an opposing second surface, the first surface being disposed adjacent to an adhesive located on the absorbent article. The release liner, the backsheet, or both may include a biodegradable film of the invention

Other features and aspects of the present invention are discussed in greater detail below.

### Brief Description of the Drawings

A full and enabling disclosure of the present invention, including the best mode thereof, directed to one of ordinary skill in the art, is set forth more particularly in the remainder of the specification, which makes reference to the appended figures in which:
Fig. 1 is a stylized illustration of one embodiment of a biodegradable film in accordance with the present invention.
Fig. 2 is a schematic illustration of one embodiment of a method for forming a biodegradable film in accordance with the present invention;
Fig. 3 is a top view of an absorbent article that may be formed in accordance with one embodiment of the present invention;
Fig. 4 is a chart of modulus test values for various samples of biodegradable films;
Fig. 5 is a chart of strain at break test values for various samples of biodegradable films;
Fig. 6 is a scanning electron micrograph (SEM) of a cross section of a biodegradable film;
Fig. 7a is back scattered electron image of a cross section of another biodegradable film;
Fig. 7b is a secondary electron image of another biodegradable film;
Fig. 8a is back scattered electron image of a cross section of a further biodegradable film;
Fig. 8b is a back scattered electron image of a cross direction section of a further biodegradable film;
Fig. 8c is a back scattered electron image of a machine direction section of a further biodegradable film;

Repeat use of references characters in the present specification and drawings is intended to represent same or analogous features or elements of the invention.

### Detailed Description of Representative Embodiments

Reference now will be made in detail to various embodiments of the invention, one or more examples of which are set forth below. Each example is provided by way of explanation of the invention, not limitation of the invention. For instance, features illustrated or described as part of one embodiment, may be used on another embodiment to yield a still further embodiment. Thus, it is intended that the present invention covers such modifications and variations as come within the scope of the appended claims.

Generally speaking, the present invention is directed to a film that is biodegradable in that it loses its integrity over time. The film contains a biodegradable polyester, oxidized starch, and plasticizer. The desired attributes of film may be achieved in the present invention by selectively controlling a variety of aspects of the film construction, such as the nature of the components employed, the relative amount of each component, the manner in which the film is formed, and so forth.

As used herein, the term "biodegradable" generally refers to a material that degrades from the action of naturally occurring microorganisms, such as bacteria, fungi, and algae; environmental heat; moisture; or other environmental factors. The biodegradability of a material is determined using ASTM Test Method 5338.92.

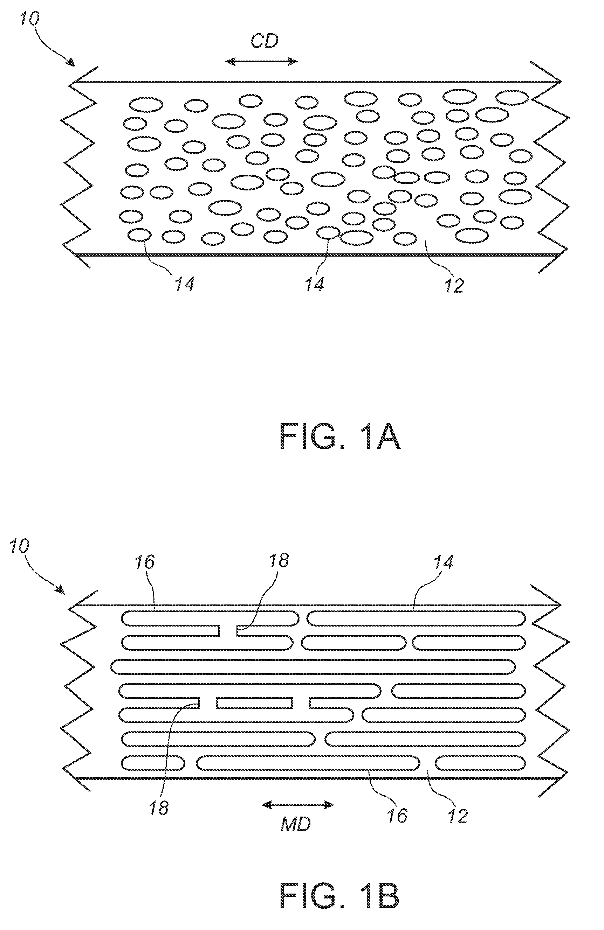

Referring to Fig. 1, a film 10 includes a first matrix phase 12 and a second dispersed phase 14. The first matrix phase 12 includes the biodegradable polyester. The second dispersed phase 14 includes the plasticized oxidized starch. The plasticized oxidized starch includes oxidized starch and plasticizer. The matrix phase comprises a smaller weight percentage of the film than does the dispersed phase.

In certain embodiments of the film 10, the dispersed phases 14 may appear as highly extended lamellar-shaped dispersed structures 16. By "highly extended" it is meant that the ratio ("L/W") of i) the largest length of a dispersed structure measured in either a cross-direction or a machine direction ("L") to ii) the largest thickness of a dispersed structure measured in the direction of the thickness of the film ("W") may range in some embodiments from about 2 to about 30, in some embodiments from about 5 to about 25, and in some embodiments from about 10 to about 20. The largest thickness of the dispersed structures "W" may range in some embodiments from about 0.01 microns to about 1 micron, in some embodiments from about .05 microns to about 0.9 microns, and in some embodiments from about 0.1 microns to about 0.8 microns. The largest thickness of the matrix phase between the dispersed structures may range in some embodiments from about 0.01 microns to about 1 micron, in some embodiments from about .05 microns to about 0.9 microns, and in some embodiments from about 0.1 microns to about 0.8 microns. The highly extended lamellar-shaped dispersed structures 16 may be interconnected by one or more bridges 18 that include the plasticized oxidized starch. The structural arrangement mimics a micro-layered film laminate in which finely divided layers of biodegradable polyester are inter-laminated between micro-layers of thermoplastic oxidized starch. Without wishing to be bound by theory, the inventors believe that the structural arrangement of the phases contributes to the good mechanical properties of the film.

In this regard, various embodiments of the present invention will now be described in more detail below.

### I. Film Components

### A. Biodegradable Polyester

The term "biodegradable" generally refers to a material that degrades from the action of naturally occurring microorganisms, such as bacteria, fungi, and algae; environmental heat; moisture; or other environmental factors, such as determined according to ASTM Test Method 5338.92. The biodegradable polyesters employed in the present invention typically have a relatively low glass transition temperature ("T_{g}") to reduce stiffness of the film and improve the processability of the polymers. For example, the T_{g} may be about 25°C or less, in some embodiments about 0°C or less, and in some embodiments, about -10°C or less. Likewise, the melting point of the biodegradable polyesters is also relatively low to improve the rate of biodegradation. For example, the melting point is typically from about 50°C to about 180°C, in some embodiments from about 80°C to about 160°C, and in some embodiments, from about 100°C to about 140°C. The melting temperature and glass transition temperature may be determined using differential scanning calorimetry ("DSC") in accordance with ASTM D-3417 as is well known in the art. Such tests may be employed using a DSC Q100 Differential Scanning Calorimeter (outfitted with a liquid nitrogen cooling accessory) and with a THERMAL ADVANTAGE (release 4.6.6) analysis software program, which are available from T.A. Instruments Inc. of New Castle, Delaware.

The biodegradable polyesters may also have a number average molecular weight ("Mₙ") ranging from about 40,000 to about 120,000 grams per mole, in some embodiments from about 50,000 to about 100,000 grams per mole, and in some embodiments, from about 60,000 to about 85,000 grams per mole. Likewise, the polyesters may also have a weight average molecular weight ("M_{w}") ranging from about 70,000 to about 300,000 grams per mole, in some embodiments from about 80,000 to about 200,000 grams per mole, and in some embodiments, from about 100,000 to about 150,000 grams per mole. The ratio of the weight average molecular weight to the number average molecular weight ("M_{w}/Mₙ"), i.e., the "polydispersity index", is also relatively low. For example, the polydispersity index typically ranges from about 1.0 to about 4.0, in some embodiments from about 1.2 to about 3.0, and in some embodiments, from about 1.4 to about 2.0. The weight and number average molecular weights may be determined by methods known to those skilled in the art.

The biodegradable polyesters may also have an apparent viscosity of from about 100 to about 1000 Pascal seconds (Pa·s), in some embodiments from about 200 to about 800 Pa·s, and in some embodiments, from about 300 to about 600 Pa·s, as determined at a temperature of 170°C and a shear rate of 1000 sec⁻¹. The melt flow index of the biodegradable polyesters may also range from about 0.1 to about 30 grams per 10 minutes, in some embodiments from about 0.5 to about 10 grams per 10 minutes, and in some embodiments, from about 1 to about 5 grams per 10 minutes. The melt flow index is the weight of a polymer (in grams) that may be forced through an extrusion rheometer orifice (2.1 mm (0.0825-inch) diameter) when subjected to a load of 2160 grams in 10 minutes at a certain temperature (e.g., 190°C), measured in accordance with ASTM Test Method D1238-E.

Of course, the melt flow index of the biodegradable polyesters will ultimately depend upon the selected film-forming process. For example, when extruded as a cast film, higher melt flow index polymers are typically desired, such as about 4 grams per 10 minutes or more, in some embodiments, from about 5 to about 12 grams per 10 minutes, and in some embodiments, from about 7 to about 9 grams per 10 minutes. Likewise, when formed as a blown film, lower melt flow index polymers are typically desired, such as less than about 12 grams per 10 minutes or less, in some embodiments from about 1 to about 7 grams per 10 minutes, and in some embodiments, from about 2 to about 5 grams per 10 minutes.

Examples of suitable biodegradable polyesters include aliphatic polyesters, such as polycaprolactone, polyesteramides, modified polyethylene terephthalate, polylactic acid (PLA) and its copolymers, terpolymers based on polylactic acid, polyglycolic acid, polyalkylene carbonates (such as polyethylene carbonate), polyhydroxyalkanoates (PHA), poly-3-hydroxybutyrate (PHB), poly-3-hydroxyvalerate (PHV), poly-3-hydroxybutyrate-co-4-hydroxybutyrate, poly-3-hydroxybutyrate-co-3-hydroxyvalerate copolymers (PHBV), poly-3-hydroxybutyrate-co-3-hydroxyhexanoate, poly-3-hydroxybutyrate-co-3-hydroxyoctanoate, poly-3-hydroxybutyrate-co-3-hydroxydecanoate, poly-3-hydroxybutyrate-co-3-hydroxyoctadecanoate, and succinate-based aliphatic polymers (e.g., polybutylene succinate, polybutylene succinate adipate, polyethylene succinate, etc.); aromatic polyesters and modified aromatic polyesters; and aliphatic-aromatic copolyesters. In one particular embodiment, the biodegradable polyester is an aliphatic-aromatic copolyester (e.g., block, random, graft, etc.). The aliphatic-aromatic copolyester may be synthesized using any known technique, such as through the condensation polymerization of a polyol in conjunction with aliphatic and aromatic dicarboxylic acids or anhydrides thereof. The polyols may be substituted or unsubstituted, linear or branched, polyols selected from polyols containing 2 to about 12 carbon atoms and polyalkylene ether glycols containing 2 to 8 carbon atoms. Examples of polyols that may be used include, but are not limited to, ethylene glycol, diethylene glycol, propylene glycol, 1,2-propanediol, 1,3-propanediol, 2,2-dimethyl-1,3-propanediol, 1,2-butanediol, 1,3-butanediol, 1,4-butanediol, 1,2-pentanediol, 1,5-pentanediol, 1,6-hexanediol, polyethylene glycol, diethylene glycol, 2,2,4-trimethyl-1,6-hexanediol, thiodiethanol, 1,3-cyclohexanedimethanol, 1,4-cyclohexanedimethanol, 2,2,4,4-tetramethyl-1,3-cyclobutanediol, cyclopentanediol, triethylene glycol, and tetraethylene glycol. Preferred polyols include 1,4-butanediol; 1,3-propanediol; ethylene glycol; 1,6-hexanediol; diethylene glycol; and 1,4-cyclohexanedimethanol.

Representative aliphatic dicarboxylic acids that may be used include substituted or unsubstituted, linear or branched, non-aromatic dicarboxylic acids selected from aliphatic dicarboxylic acids containing 2 to about 10 carbon atoms, and derivatives thereof. Non-limiting examples of aliphatic dicarboxylic acids include malonic, malic, succinic, oxalic, glutaric, adipic, pimelic, azelaic, sebacic, fumaric, 2,2-dimethyl glutaric, suberic, 1,3-cyclopentanedicarboxylic, 1,4-cyclohexanedicarboxylic, 1,3-cyclohexanedicarboxylic, diglycolic, itaconic, maleic, and 2,5-norbornanedicarboxylic acids. Representative aromatic dicarboxylic acids that may be used include substituted and unsubstituted, linear or branched, aromatic dicarboxylic acids selected from aromatic dicarboxylic acids containing 8 or more carbon atoms, and derivatives thereof. Non-limiting examples of aromatic dicarboxylic acids include terephthalic acid, dimethyl terephthalate, isophthalic acid, dimethyl isophthalate, 2,6-napthalene dicarboxylic acid, dimethyl-2,6-naphthalate, 2,7-naphthalenedicarboxylic acid, dimethyl-2,7-naphthalate, 3,4'-diphenyl ether dicarboxylic acid, dimethyl-3,4'diphenyl ether dicarboxylate, 4,4'-diphenyl ether dicarboxylic acid, dimethyl-4,4'-diphenyl ether dicarboxylate, 3,4'-diphenyl sulfide dicarboxylic acid, dimethyl-3,4'-diphenyl sulfide dicarboxylate, 4,4'-diphenyl sulfide dicarboxylic acid, dimethyl-4,4'-diphenyl sulfide dicarboxylate, 3,4'-diphenyl sulfone dicarboxylic acid, dimethyl-3,4'-diphenyl sulfone dicarboxylate, 4,4'-diphenyl sulfone dicarboxylic acid, dimethyl-4,4'-diphenyl sulfone dicarboxylate, 3,4'-benzophenonedicarboxylic acid, dimethyl-3,4'-benzophenonedicarboxylate, 4,4'-benzophenonedicarboxylic acid, dimethyl-4,4'-benzophenonedicarboxylate, 1,4-naphthalene dicarboxylic acid, dimethyl-1,4-naphthalate, 4,4'-methylene bis(benzoic acid), dimethyl-4,4'-methylenebis(benzoate), etc., and mixtures thereof.

The polymerization may be catalyzed by a catalyst, such as a titanium-based catalyst (e.g., tetraisopropyltitanate, tetraisopropoxy titanium, dibutoxydiacetoacetoxy titanium, or tetrabutyltitanate). If desired, a diisocyanate chain extender may be reacted with the copolyester to increase its molecular weight. Representative diisocyanates may include toluene 2,4-diisocyanate, toluene 2,6-diisocyanate, 2,4'-diphenylmethane diisocyanate, naphthylene-1,5-diisocyanate, xylylene diisocyanate, hexamethylene diisocyanate ("HMDI"), isophorone diisocyanate and methylenebis(2-isocyanatocyclohexane). Trifunctional isocyanate compounds may also be employed that contain isocyanurate and/or biurea groups with a functionality of not less than three, or to replace the diisocyanate compounds partially by tri-or polyisocyanates. The preferred diisocyanate is hexamethylene diisocyanate. The amount of the chain extender employed is typically from about 0.3 to about 3.5 wt.%, in some embodiments, from about 0.5 to about 2.5 wt.% based on the total weight percent of the polymer.

The copolyesters may either be a linear polymer or a long-chain branched polymer. Long-chain branched polymers are generally prepared by using a low molecular weight branching agent, such as a polyol, polycarboxylic acid, hydroxy acid, and so forth. Representative low molecular weight polyols that may be employed as branching agents include glycerol, trimethylolpropane, trimethylolethane, polyethertriols, 1,2,4-butanetriol, pentaerythritol, 1,2,6-hexanetriol, sorbitol, 1,1,4,4,-tetrakis (hydroxymethyl) cyclohexane, tris(2-hydroxyethyl) isocyanurate, and dipentaerythritol. Representative higher molecular weight polyols (molecular weight of 400 to 3000) that may be used as branching agents include triols derived by condensing alkylene oxides having 2 to 3 carbons, such as ethylene oxide and propylene oxide with polyol initiators. Representative polycarboxylic acids that may be used as branching agents include hemimellitic acid, trimellitic (1,2,4-benzenetricarboxylic) acid and anhydride, trimesic (1,3,5-benzenetricarboxylic) acid, pyromellitic acid and anhydride, benzenetetracarboxylic acid, benzophenone tetracarboxylic acid, 1,1,2,2-ethane-tetracarboxylic acid, 1,1,2-ethanetricarboxylic acid, 1,3,5-pentanetricarboxylic acid, and 1,2,3,4-cyclopentanetetracarboxylic acid. Representative hydroxy acids that may be used as branching agents include malic acid, citric acid, tartaric acid, 3-hydroxyglutaric acid, mucic acid, trihydroxyglutaric acid, 4-carboxyphthalic anhydride, hydroxyisophthalic acid, and 4-(beta-hydroxyethyl)phthalic acid. Such hydroxy acids contain a combination of 3 or more hydroxyl and carboxyl groups. Especially preferred branching agents include trimellitic acid, trimesic acid, pentaerythritol, trimethylol propane and 1,2,4-butanetriol.

The aromatic dicarboxylic acid monomer constituent may be present in the copolyester in an amount of from about 10 mole% to about 40 mole%, in some embodiments from about 15 mole% to about 35 mole%, and in some embodiments, from about 15 mole% to about 30 mole%. The aliphatic dicarboxylic acid monomer constituent may likewise be present in the copolyester in an amount of from about 15 mole% to about 45 mole%, in some embodiments from about 20 mole% to about 40 mole%, and in some embodiments, from about 25 mole% to about 35 mole%. The polyol monomer constituent may also be present in the aliphatic-aromatic copolyester in an amount of from about 30 mole% to about 65 mole%, in some embodiments from about 40 mole% to about 50 mole%, and in some embodiments, from about 45 mole% to about 55 mole%.

In one particular embodiment, for example, the aliphatic-aromatic copolyester may comprise the following structure: 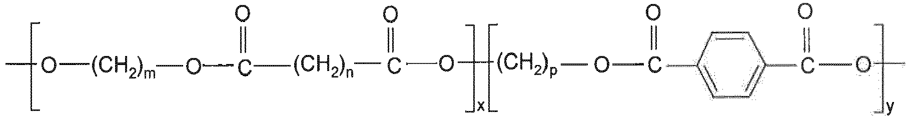 wherein,
m is an integer from 2 to 10, in some embodiments from 2 to 4, and in one embodiment, 4;
n is an integer from 0 to 18, in some embodiments from 2 to 4, and in one embodiment, 4;
p is an integer from 2 to 10, in some embodiments from 2 to 4, and in one embodiment, 4;
x is an integer greater than 1; and
y is an integer greater than 1. One example of such a copolyester is polybutylene adipate terephthalate, which is commercially available under the designation ECOFLEX® F BX 7011 from BASF Corp. Another example of a suitable copolyester containing an aromatic terephthalic acid monomer constituent is available under the designation ENPOL™ 8060M from IRE Chemicals (South Korea). Other suitable aliphatic-aromatic copolyesters may be described in U.S. Patent Nos. 5,292,783; 5,446,079; 5,559,171; 5,580,911; 5,599,858; 5,817,721; 5,900,322; and 6,258,924.

### B. Oxidized Starch

An oxidized starch is also employed that is biodegradable in that it contains one or more oxidized starches that are generally biodegradable. Starch is a natural polymer composed of amylose and amylopectin. Amylose is essentially a linear polymer having a molecular weight in the range of 100,000-500,000, whereas amylopectin is a highly branched polymer having a molecular weight of up to several million. Although starch is produced in many plants, typical sources includes seeds of cereal grains, such as corn, waxy corn, wheat, sorghum, rice, and waxy rice; tubers, such as potatoes; roots, such as tapioca (i.e., cassava and manioc), sweet potato, and arrowroot; and the pith of the sago palm. Any natural (unmodified) and/or modified wheat or corn starch may be oxidized for use in the present invention. Oxidation of starches may be accomplished by treating the starch with oxidants. These oxidants include, but are not limited to, hydrochloric acid, sodium hypochlorite, calcium hypochlorite, and so forth. Chemical oxidation of starch alters hydrogen bonding by creating carbonyl and carboxyl functional groups on starch backbones. Even when starch oxidation is at a low level of intensity, an improved interaction with synthetic polymers is expected, particularly with aliphatic and aromatic copolyesters, because of molecular structural (polar) similarities in carbonyl or carboxyl and repeating glucose units and the ester groups in copolyesters. Material aggregation in the blend is significantly reduced as a result of the better interfacial adhesion through the polar interactions between the carboxyl and carbonyl groups on oxidized starch and polar ester groups on copolyesters. Carbonyl and carboxyl groups may also form a low level of cross-linking, which further enhances mechanical performance of the blend even at a high levels of TPOS incorporation.

Modified starches that have been chemically modified by other typical processes known in the art (e.g., esterification, etherification, acid hydrolysis, enzymatic hydrolysis, etc.) are often employed to be oxidized. Starch ethers and/or esters may be particularly desirable, such as hydroxyalkyl starches, carboxymethyl starches, etc. The hydroxyalkyl group of hydroxylalkyl starches may contain, for instance, 2 to 10 carbon atoms, in some embodiments from 2 to 6 carbon atoms, and in some embodiments, from 2 to 4 carbon atoms. Representative hydroxyalkyl starches such as hydroxyethyl starch, hydroxypropyl starch, hydroxybutyl starch, and derivatives thereof. Starch esters, for instance, may be prepared using a wide variety of anhydrides (e.g., acetic, propionic, butyric, and so forth), organic acids, acid chlorides, or other esterification reagents. The degree of esterification may vary as desired, such as from 1 to 3 ester groups per glucosidic unit of the starch.

### C. Plasticizer

A plasticizer is also employed in the film to help render the biodegradable polyester and/or starch melt-processable. Starches, for instance, normally exist in the form of granules that have a coating or outer membrane that encapsulates the more amylose and amylopectin chains within the interior of the granule. When heated, plasticizers (e.g., polar solvents) may soften and penetrate the outer membrane and cause the inner starch chains to absorb water and swell. This swelling will, at some point, cause the outer shell to rupture and result in an irreversible destructurization of the starch granule. Once destructurized, the starch polymer chains containing amylose and amylopectin polymers, which are initially compressed within the granules, will stretch out and form a generally disordered intermingling of polymer chains. Upon resolidification, however, the chains may reorient themselves to form crystalline or amorphous solids having varying strengths depending on the orientation of the starch polymer chains. Because the starch (natural or modified) is thus capable of melting and resolidifying, it is generally considered a "thermoplastic starch."

Suitable plasticizers may include, for instance, polyhydric alcohol plasticizers, such as sugars (e.g., glucose, sucrose, fructose, raffinose, maltodextrose, galactose, xylose, maltose, lactose, mannose, and erythrose), sugar alcohols (e.g., erythritol, xylitol, malitol, mannitol, and sorbitol), polyols (e.g., ethylene glycol, glycerol (glycerin), propylene glycol, dipropylene glycol, butylene glycol, and hexane triol), etc. Also suitable are hydrogen bond forming organic compounds which do not have hydroxyl group, including urea and urea derivatives; anhydrides of sugar alcohols such as sorbitan; animal proteins such as gelatin; vegetable proteins such as sunflower protein, soybean proteins, cotton seed proteins; and mixtures thereof. Other suitable plasticizers may include phthalate esters, dimethyl and diethylsuccinate and related esters, glycerol triacetate, glycerol mono and diacetates, glycerol mono, di, and tripropionates, butanoates, stearates, lactic acid esters, citric acid esters, adipic acid esters, stearic acid esters, oleic acid esters, and other acid esters. Aliphatic acids may also be used, such as ethylene acrylic acid, ethylene maleic acid, butadiene acrylic acid, butadiene maleic acid, propylene acrylic acid, propylene maleic acid, and other hydrocarbon based acids. A low molecular weight plasticizer is preferred, such as less than about 20,000 g/mol, preferably less than about 5,000 g/mol and more preferably less than about 1,000 g/mol.

The plasticizer may be incorporated into the film using any of a variety of known techniques. For example, the starch may be "pre-plasticized" prior to incorporation into the film. Alternatively, one or more of the components may be plasticized at the same time as they are blended together. Regardless, batch and/or continuous melt blending techniques may be employed to blend the components. For example, a mixer/kneader, Banbury mixer, Farrel continuous mixer, single-screw extruder, twin-screw extruder, roll mill, etc., may be utilized. One particularly suitable melt-blending device is a co-rotating, twin-screw extruder (e.g., USALAB twin-screw extruder available from Thermo Electron Corporation of Stone, England or an extruder available from Werner-Pfreiderer from Ramsey, New Jersey). Such extruders may include feeding and venting ports and provide high intensity distributive and dispersive mixing. For example, a starch composition may be initially fed to a feeding port of the twin-screw extruder. Thereafter, a plasticizer may be injected into the starch composition. Alternatively, the starch composition may be simultaneously fed to the feed throat of the extruder or separately at a different point along its length. Melt blending may occur at any of a variety of temperatures, such as from about 30°C to about 200°C, in some embodiments, from about 40°C to about 160°C, and in some embodiments, from about 50°C to about 150°C.

The amounts of the biodegradable polyester, oxidized starch, and plasticizer employed in the film are controlled in the present invention to achieve a desirable balance between biodegradability and mechanical strength. Biodegradable polyesters constitute from 1 wt.% to 49 wt.%, in some embodiments from 10 wt.% to 46 wt.%, and in some embodiments, from 20 to 43 wt.% of the film. Plasticizers may constitute from 0.1 wt.% to 40 wt.%, in some embodiments from 1 wt.% to 35 wt.%, and in some embodiments, from 5 to 30 wt.% of the film. Further, oxidized corn or wheat starches may constitute from 45 wt.% to 75 wt.%, in some embodiments from 45 wt.% to 65 wt. %, in some embodiments from 45 wt. % to 55 wt. %, in some embodiments from 47 wt.% to 75 wt.%, in some embodiments from 47 wt. % to 65 wt. %, in some embodiments from 47 wt. % to 55 wt. %, in some embodiments from 50 wt. % to 75 wt.%, in some embodiments from 50 wt. % to 65 wt. %, and in some embodiments from 50 wt. % to 55 wt. % of the film. It should be understood that the weight of oxidized starch referenced herein includes any bound water that naturally occurs in the oxidized starch before mixing it with other components. Oxidized starches, for instance, typically have a bound water content of about 5% to about 16% by weight of the starch.

In some embodiments the oxidized corn or wheat starch and a plasticizer are separately dispersively blended to form a thermoplastic oxidized corn or wheat starch. The thermoplastic oxidized starch constitutes from 46 wt. % to 75 wt. %, in some embodiments from 50 wt. % to 70 wt. %, in some embodiments from 50 wt. % to 65 wt. %, in some embodiments from 55 wt. % to 70 wt. %, and in some embodiments from 55 wt. % to 65 wt. % of the film.

### D. Other Components

In addition to the components noted above, other additives may also be incorporated into the film of the present invention, such as dispersion aids, melt stabilizers, processing stabilizers, heat stabilizers, light stabilizers, antioxidants, heat aging stabilizers, whitening agents, antiblocking agents, bonding agents, lubricants, water soluble polymers, fillers, etc. Dispersion aids, for instance, may also be employed to help create a uniform dispersion of the starch/polyvinyl alcohol/plasticizer mixture and retard or prevent separation into constituent phases. Likewise, the dispersion aids may also improve the water dispersibility of the film. When employed, the dispersion aid(s) typically constitute from about 0.01 wt.% to about 15 wt.%, in some embodiments from about 0.1 wt.% to about 10 wt.%, and in some embodiments, from about 0.5 wt.% to about 5 wt.% of the film. Although any dispersion aid may generally be employed in the present invention, surfactants having a certain hydrophilic/lipophilic balance ("HLB") may improve the long-term stability of the composition. The HLB index is well known in the art and is a scale that measures the balance between the hydrophilic and lipophilic solution tendencies of a compound. The HLB scale ranges from 1 to approximately 50, with the lower numbers representing highly lipophilic tendencies and the higher numbers representing highly hydrophilic tendencies. In some embodiments of the present invention, the HLB value of the surfactants is from about 1 to about 20, in some embodiments from about 1 to about 15 and in some embodiments, from about 2 to about 10. If desired, two or more surfactants may be employed that have HLB values either below or above the desired value, but together have an average HLB value within the desired range.

One particularly suitable class of surfactants for use in the present invention are nonionic surfactants, which typically have a hydrophobic base (e.g., long chain alkyl group or an alkylated aryl group) and a hydrophilic chain (e.g., chain containing ethoxy and/or propoxy moieties). For instance, some suitable nonionic surfactants that may be used include, but are not limited to, ethoxylated alkylphenols, ethoxylated and propoxylated fatty alcohols, polyethylene glycol ethers of methyl glucose, polyethylene glycol ethers of sorbitol, ethylene oxide-propylene oxide block copolymers, ethoxylated esters of fatty (C₈ -C₁₈) acids, condensation products of ethylene oxide with long chain amines or amides, condensation products of ethylene oxide with alcohols, fatty acid esters, monoglyceride or diglycerides of long chain alcohols, and mixtures thereof. In one particular embodiment, the nonionic surfactant may be a fatty acid ester, such as a sucrose fatty acid ester, glycerol fatty acid ester, propylene glycol fatty acid ester, sorbitan fatty acid ester, pentaerythritol fatty acid ester, sorbitol fatty acid ester, and so forth. The fatty acid used to form such esters may be saturated or unsaturated, substituted or unsubstituted, and may contain from 6 to 22 carbon atoms, in some embodiments from 8 to 18 carbon atoms, and in some embodiments, from 12 to 14 carbon atoms. In one particular embodiment, mono- and di-glycerides of fatty acids may be employed in the present invention.

One or more water-soluble polymers may also be employed in the present invention. Water-soluble polymers may constitute from about 0.1 wt.% to about 40 wt.%, in some embodiments from about 1 wt.% to about 35 wt.%, and in some embodiments, from about 5 to about 30 wt.% of the film. Without intending to be limited by theory, the present inventors believe that such polymers may improve the compatibility between the starch and biodegradable polyester, thereby leading to a film that exhibits excellent mechanical and physical properties during use. Such polymers may be formed from monomers such as vinyl pyrrolidone, hydroxyethyl acrylate or methacrylate (e.g., 2-hydroxyethyl methacrylate), hydroxypropyl acrylate or methacrylate, acrylic or methacrylic acid, acrylic or methacrylic esters or vinyl pyridine, acrylamide, vinyl acetate, vinyl alcohol, ethylene oxide, derivatives thereof, and so forth. Other examples of suitable monomers are described in U.S. Patent Nos. 4,499,154 to James, et al.. The resulting polymers may be homopolymers or interpolymers (e.g., copolymer, terpolymer, etc.), and may be nonionic, anionic, cationic, or amphoteric. In addition, the polymer may be of one type (i.e., homogeneous), or mixtures of different polymers may be used (i.e., heterogeneous).

In one particular embodiment, the water-soluble polymer may contain a repeating unit having a functional hydroxyl group, such as polyvinyl alcohol ("PVOH"), copolymers of polyvinyl alcohol (e.g., ethylene vinyl alcohol copolymers, methyl methacrylate vinyl alcohol copolymers, etc.), etc. Vinyl alcohol polymers, for instance, have at least two or more vinyl alcohol units in the molecule and may be a homopolymer of vinyl alcohol, or a copolymer containing other monomer units. Vinyl alcohol homopolymers may be obtained by hydrolysis of a vinyl ester polymer, such as vinyl formate, vinyl acetate, vinyl propionate, etc. Vinyl alcohol copolymers may be obtained by hydrolysis of a copolymer of a vinyl ester with an olefin having 2 to 30 carbon atoms, such as ethylene, propylene, 1-butene, etc.; an unsaturated carboxylic acid having 3 to 30 carbon atoms, such as acrylic acid, methacrylic acid, crotonic acid, maleic acid, fumaric acid, etc., or an ester, salt, anhydride or amide thereof; an unsaturated nitrile having 3 to 30 carbon atoms, such as acrylonitrile, methacrylonitrile, etc.; a vinyl ether having 3 to 30 carbon atoms, such as methyl vinyl ether, ethyl vinyl ether, etc.; and so forth. The degree of hydrolysis may be selected to optimize solubility, etc., of the polymer. For example, the degree of hydrolysis may be from about 60 mole% to about 95 mole%, in some embodiments from about 80 mole% to about 90 mole%, and in some embodiments, from about 85 mole% to about 89 mole%. Examples of suitable partially hydrolyzed polyvinyl alcohol polymers are available under the designation CELVOL™ 203, 205, 502, 504, 508, 513, 518, 523, 530, or 540 from Celanese Corp. Other suitable partially hydrolyzed polyvinyl alcohol polymers are available under the designation ELVANOL™ 50-14, 50-26, 50-42, 51-03, 51-04, 51-05, 51-08, and 52-22 from DuPont.

Fillers may also be employed in the present invention. Fillers are particulates or other forms of material that may be added to the film polymer extrusion blend and that will not chemically interfere with the extruded film, but which may be uniformly dispersed throughout the film. Fillers may serve a variety of purposes, including enhancing film opacity and/or breathability (i.e., vapor-permeable and substantially liquid-impermeable). For instance, filled films may be made breathable by stretching, which causes the polymer to break away from the filler and create microporous passageways. Breathable microporous elastic films are described, for example, in U.S. Patent Nos. 5,997,981; 6,015,764; and 6,111,163 to McCormack, et al.; 5,932,497 to Morman, et al.; 6,461,457 to Taylor, et al., Further, hindered phenols are commonly used as an antioxidant in the production of films. Some suitable hindered phenols include those available from Ciba Specialty Chemicals under the trade name "Irganox®", such as Irganox® 1076, 1010, or E 201. Moreover, bonding agents may also be added to the film to facilitate bonding of the film to additional materials (e.g., nonwoven webs). Examples of such bonding agents include hydrogenated hydrocarbon resins. Other suitable bonding agents are described in U.S. Patent Nos. 4,789,699 to Kieffer et al. and 5,695,868 to McCormack.

### II. Film Construction

The film of the present invention may be mono- or multi-layered. Multilayer films may be prepared by co-extrusion of the layers, extrusion coating, or by any conventional layering process. Such multilayer films normally contain at least one base layer and at least one skin layer, but may contain any number of layers desired. For example, the multilayer film may be formed from a base layer and one or more skin layers, wherein the base layer is formed from a blend of the biodegradable polyester, starch, and plasticizer. In most embodiments, the skin layer(s) are formed from a biodegradable polyester, thermoplastic starch, and plasticizer as described above. It should be understood, however, that other polymers may also be employed in the skin layer(s), such as polyolefin polymers (e.g., linear low-density polyethylene (LLDPE) or polypropylene). The term "linear low density polyethylene" refers to polymers of ethylene and higher alpha olefin comonomers, such as C₃- C₁₂ and combinations thereof, having a Melt Index (as measured by ASTM D-1238) of from about 0.5 to about 30 grams per 10 minutes at 190°C. Examples of predominately linear polyolefin polymers include, without limitation, polymers produced from the following monomers: ethylene, propylene, 1-butene, 4-methyl-pentene, 1-hexene, 1-octene and higher olefins as well as copolymers and terpolymers of the foregoing. In addition, copolymers of ethylene and other olefins including butene, 4-methyl-pentene, hexene, heptene, octene, decene, etc., are also examples of predominately linear polyolefin polymers. Additional film-forming polymers that may be suitable for use with the present invention, alone or in combination with other polymers, include ethylene vinyl acetate, ethylene ethyl acrylate, ethylene acrylic acid, ethylene methyl acrylate, ethylene normal butyl acrylate, nylon, ethylene vinyl alcohol, polystyrene, polyurethane, and so forth.

Any known technique may be used to form a film from the compounded material, including blowing, casting, flat die extruding, etc. In one particular embodiment, the film may be formed by a blown process in which a gas (e.g., air) is used to expand a bubble of the extruded polymer blend through an annular die. The bubble is then collapsed and collected in flat film form. Processes for producing blown films are described, for instance, in U.S. Patent Nos. 3,354,506 to Raley; 3,650,649 to Schippers; and 3,801,429 to Schrenk et al., as well as U.S. Patent Application Publication Nos. 2005/0245162 to McCormack, et al. and 2003/0068951 to Boggs, et al.. In yet another embodiment, however, the film is formed using a casting technique.

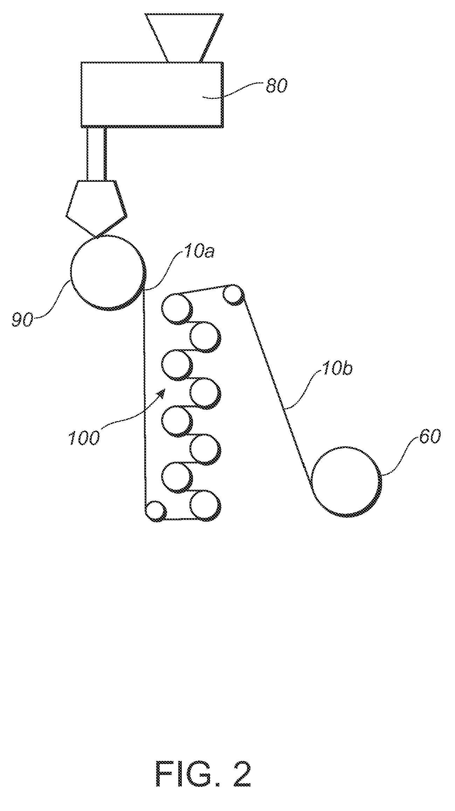

Referring to Fig. 2, for instance, one embodiment of a method for forming a cast film is shown. The raw materials (e.g., biodegradable polyester, oxidized starch, plasticizer, etc.) may be supplied to a melt blending device, either separately or as one or more blends. In one embodiment, for example, an oxidized starch and a plasticizer are separately supplied to a melt blending device where they are dispersively blended in a manner such as described above to form a thermoplastic oxidized starch For example, an extruder may be employed that includes feeding and venting ports. In one embodiment, the biodegradable polyester may be fed to a feeding port of the twin-screw extruder and melted. Thereafter, the thermoplastic oxidized starch may be fed into the polymer melt. Regardless, the materials are blended under high shear/pressure and heat to ensure sufficient mixing. For example, melt blending may occur at a temperature of from about 50°C to about 300°C, in some embodiments, from about 70°C to about 250°C, and in some embodiments, from about 90°C to about 180°C. Likewise, the apparent shear rate during melt blending may range from about 100 seconds⁻¹ to about 10,000 seconds⁻¹, in some embodiments from about 500 seconds⁻¹ to about 5000 seconds⁻¹, and in some embodiments, from about 800 seconds⁻¹ to about 1200 seconds⁻¹. The apparent shear rate is equal to *4*Q/π *R³*, where Q is the volumetric flow rate ("m³/s") of the polymer melt and R is the radius ("m") of the capillary (e.g., extruder die) through which the melted polymer flows.

Thereafter, the extruded material may be immediately chilled and cut into pellet form. In the particular embodiment of Fig. 2, the compounded material (not shown) is then supplied to an extrusion apparatus 80 and cast onto a casting roll 90 to form a single-layered precursor film 10a. If a multilayered film is to be produced, the multiple layers are co-extruded together onto the casting roll 90. The casting roll 90 may optionally be provided with embossing elements to impart a pattern to the film. Typically, the casting roll 90 is kept at temperature sufficient to solidify and quench the sheet 10a as it is formed, such as from about 20 to 60°C. If desired, a vacuum box may be positioned adjacent to the casting roll 90 to help keep the precursor film 10a close to the surface of the roll 90. Additionally, air knives or electrostatic pinners may help force the precursor film 10a against the surface of the casting roll 90 as it moves around a spinning roll. An air knife is a device known in the art that focuses a stream of air at a very high flow rate to pin the edges of the film.

Once cast, the film 10a may then be optionally oriented in one or more directions to further improve film uniformity and reduce thickness. Orientation may also form micropores in a film containing a filler, thus providing breathability to the film. For example, the film may be immediately reheated to a temperature below the melting point of one or more polymers in the film, but high enough to enable the composition to be drawn or stretched. In the case of sequential orientation, the "softened" film is drawn by rolls rotating at different speeds of rotation such that the sheet is stretched to the desired draw ratio in the longitudinal direction (machine direction). This "uniaxially" oriented film may then be laminated to a fibrous web. In addition, the uniaxially oriented film may also be oriented in the cross-machine direction to form a "biaxially oriented" film. For example, the film may be clamped at its lateral edges by chain clips and conveyed into a tenter oven. In the tenter oven, the film may be reheated and drawn in the cross-machine direction to the desired draw ratio by chain clips diverged in their forward travel.

Referring again to Fig. 2, for instance, one method for forming a uniaxially oriented film is shown. As illustrated, the precursor film 10a is directed to a film-orientation unit 100 or machine direction orienter ("MDO"), such as commercially available from Marshall and Willams, Co. of Providence, Rhode Island. The MDO has a plurality of stretching rolls (such as from 5 to 8) which progressively stretch and thin the film in the machine direction, which is the direction of travel of the film through the process as shown in Fig. 2. While the MDO 100 is illustrated with eight rolls, it should be understood that the number of rolls may be higher or lower, depending on the level of stretch that is desired and the degrees of stretching between each roll. The film may be stretched in either single or multiple discrete stretching operations. It should be noted that some of the rolls in an MDO apparatus may not be operating at progressively higher speeds. If desired, some of the rolls of the MDO 100 may act as preheat rolls. If present, these first few rolls heat the film 10a above room temperature (e.g., to 125°F). The progressively faster speeds of adjacent rolls in the MDO act to stretch the film 10a. The rate at which the stretch rolls rotate determines the amount of stretch in the film and final film weight.

The resulting film 10b may then be wound and stored on a take-up roll 60. While not shown here, various additional potential processing and/or finishing steps known in the art, such as slitting, treating, aperturing, printing graphics, or lamination of the film with other layers (e.g., nonwoven web materials), may be performed without departing from the scope of the invention.

The thickness of the resulting biodegradable film may generally vary depending upon the desired use. Nevertheless, the film thickness is typically minimized to reduce the time needed for the film to biodegrade. Thus, in most embodiments of the present invention, the biodegradable film has a thickness of about 50 micrometers or less, in some embodiments from about 1 to about 40 micrometers, in some embodiments from about 2 to about 35 micrometers, and in some embodiments, from about 5 to about 30 micrometers.

Despite having such a small thickness, the film of the present invention is nevertheless able to retain good dry mechanical properties during use. One parameter that is indicative of the relative dry strength of the film is the ultimate tensile strength, which is equal to the peak stress obtained in a stress-strain curve. Desirably, the film of the present invention exhibits an ultimate tensile strength in the machine direction ("MD") of from about 10 to about 80 Megapascals (MPa), in some embodiments from about 10 to about 60 MPa, and in some embodiments, from about 10 to about 50 MPa, and an ultimate tensile strength in the cross-machine direction ("CD") of from about 2 to about 40 Megapascals (MPa), in some embodiments from about 4 to about 40 MPa, and in some embodiments, from about 5 to about 30 MPa. Although possessing good strength, it is also desirable that the film is not too stiff. One parameter that is indicative of the relative stiffness of the film (when dry) is Young's modulus of elasticity, which is equal to the ratio of the tensile stress to the tensile strain and is determined from the slope of a stress-strain curve. For example, the film typically exhibits a Young's modulus in the machine direction ("MD") of from about 50 to about 200 Megapascals ("MPa"), in some embodiments from about 50 to about 100 MPa, and in some embodiments, from about 60 to about 80 MPa, and a Young's modulus in the cross-machine direction ("CD") of from about 50 to about 200 Megapascals ("MPa"), in some embodiments from about 50 to about 100 MPa, and in some embodiments, from about 60 to about 80 MPa. The MD and CD elongation of the film, respectively, may also be about 100% or more, in some embodiments about 200% or more, and in some embodiments, about 300% or more, in some embodiments about 400% or more, and in some embodiments about 500% or more.

### III. Articles

The biodegradable film of the present invention may be used in a wide variety of applications. For example, as indicated above, the film may be used in an absorbent article. An "absorbent article" generally refers to any article capable of absorbing water or other fluids. Examples of some absorbent articles include, but are not limited to, personal care absorbent articles, such as diapers, training pants, absorbent underpants, incontinence articles, feminine hygiene products (e.g., sanitary napkins, pantiliners, etc.), swim wear, baby wipes, and so forth; medical absorbent articles, such as garments, fenestration materials, underpads, bedpads, bandages, absorbent drapes, and medical wipes; food service wipers; clothing articles; and so forth. Several examples of such absorbent articles are described in U.S. Patent Nos. 5,649,916 to DiPalma, et al.; 6,110,158 to Kielpikowski; 6,663,611 to Blarney, et al., which are incorporated herein in their entirety by reference thereto for all purposes. Still other suitable articles are described in U.S. Patent Application Publication No. 2004/0060112 A1 to Fell et al., as well as U.S. Patent Nos. 4,886,512 to Damico et al.; 5,558,659 to Sherrod et al.; 6,888,044 to Fell et al.; and 6,511,465 to Freiburger et al.. Materials and processes suitable for forming such absorbent articles are well known to those skilled in the art.

As is well known in the art, the absorbent article may be provided with adhesives (e.g., pressure-sensitive adhesives) that help removably secure the article to the crotch portion of an undergarment and/or wrap up the article for disposal. Suitable pressure-sensitive adhesives, for instance, may include acrylic adhesives, natural rubber adhesives, tackified block copolymer adhesives, polyvinyl acetate adhesives, ethylene vinyl acetate adhesives, silicone adhesives, polyurethane adhesives, thermosettable pressure-sensitive adhesives, such as epoxy acrylate or epoxy polyester pressure-sensitive adhesives, etc. Such pressure-sensitive adhesives are known in the art and are described in the Handbook of Pressure Sensitive Adhesive Technology, Satas (Donatas), 1989, 2nd edition, Van Nostrand Reinhold. The pressure sensitive adhesives may also include additives such as cross-linking agents, fillers, gases, blowing agents, glass or polymeric microspheres, silica, calcium carbonate fibers, surfactants, and so forth. The additives are included in amounts sufficient to affect the desired properties.

The location of the adhesive on the absorbent article is not critical and may vary widely depending on the intended use of the article. For example, certain feminine hygiene products (e.g., sanitary napkins) may have wings or flaps that laterally from a central absorbent core and are intended to be folded around the edges of the wearer's panties in the crotch region. The flaps may be provided with an adhesive (e.g., pressure-sensitive adhesive) for affixing the flaps to the underside of the wearer's panties.

Regardless of the particular location of the adhesive, however, a release liner may be employed to cover the adhesive, thereby protecting it from dirt, drying out, and premature sticking prior to use. The release liner may contain a release coating that enhances the ability of the liner to be peeled from an adhesive. The release coating contains a release agent, such as a hydrophobic polymer. Exemplary hydrophobic polymers include, for instance, silicones (e.g., polysiloxanes, epoxy silicones, etc.), perfluoroethers, fluorocarbons, polyurethanes, and so forth. Examples of such release agents are described, for instance, in U.S. Patent Nos. 6,530,910 to Pomplun, et al.; 5,985,396 to Kerins, et al.; and 5,981,012 to Pomplun, et al.. One particularly suitable release agent is an amorphous polyolefin having a melt viscosity of about 400 to about 10,000 cps (about 400 to about 10,000 mPa·s) at 190°C, such as made by the U.S. Rexene Company under the tradename REXTAC® (e.g., RT2315, RT2535 and RT2330). The release coating may also contain a detackifier, such as a low molecular weight, highly branched polyolefin. A particularly suitable low molecular weight, highly branched polyolefin is VYBAR® 253, which is made by the Petrolite Corporation. Other additives may also be employed in the release coating, such as compatibilizers, processing aids, plasticizers, tackifiers, slip agents, and antimicrobial agents, and so forth. The release coating may be applied to one or both surfaces of the liner, and may cover all or only a portion of a surface. Any suitable technique may be employed to apply the release coating, such as solvent-based coating, hot melt coating, solventless coating, etc. Solvent-based coatings are typically applied to the release liner by processes such as roll coating, knife coating, curtain coating, gravure coating, wound rod coating, and so forth. The solvent (e.g., water) is then removed by drying in an oven, and the coating is optionally cured in the oven. Solventless coatings may include solid compositions, such as silicones or epoxy silicones, which are coated onto the liner and then cured by exposure to ultraviolet light. Optional steps include priming the liner before coating or surface modification of the liner, such as with corona treatment. Hot melt coatings, such as polyethylenes or perfluoroethers, may be heated and then applied through a die or with a heated knife. Hot melt coatings may be applied by co-extruding the release agent with the release liner in blown film or sheet extruder for ease of coating and for process efficiency.

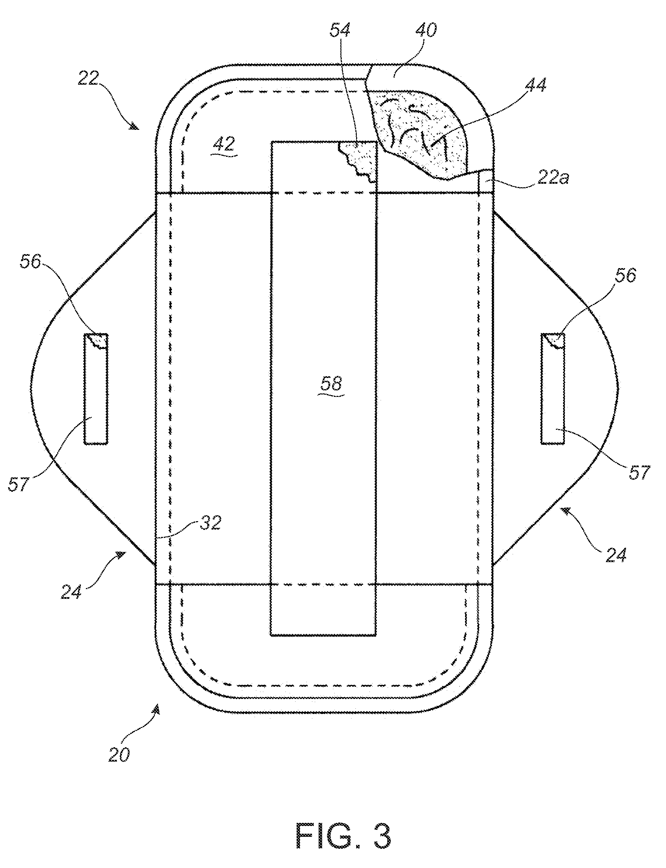

To facilitate their ability to be easily disposed, various components of an absorbent article may be formed from a biodegradable film in accordance with the present invention. For example, films formed from a biodegradable film in accordance with the present invention may be useful as baffle (backsheet) films for adult and feminine pads and liners, outercover films for diapers and training pants, packaging films for product bags containing disposable garment products, and so forth. In this regard, one particular embodiment of a sanitary napkin that may employ the biodegradable film of the present invention will now be described in more detail. For purposes of illustration only, an absorbent article 20 is shown in Fig. 3 as a sanitary napkin for feminine hygiene. However, as noted above, the invention may be embodied in other types of absorbent articles, such as incontinence articles, diapers, diaper pants, children's training pants, and so forth. In the illustrated embodiment, the absorbent article 20 includes a main body portion 22 containing a topsheet 40, an outer cover or backsheet 42, an absorbent core 44 positioned between the backsheet 42 and the topsheet 40, and a pair of flaps 24 extending from each longitudinal side 22a of the main body portion 22. The topsheet 40 defines a bodyfacing surface of the absorbent article 20. The absorbent core 44 is positioned inward from the outer periphery of the absorbent article 20 and includes a body-facing side positioned adjacent the topsheet 40 and a garment-facing surface positioned adjacent the backsheet 42.

The topsheet 40 is generally designed to contact the body of the user and is liquid-permeable. The topsheet 40 may surround the absorbent core 44 so that it completely encases the absorbent article 20. Alternatively, the topsheet 40 and the backsheet 42 may extend beyond the absorbent core 44 and be peripherally joined together, either entirely or partially, using known techniques. Typically, the topsheet 40 and the backsheet 42 are joined by adhesive bonding, ultrasonic bonding, or any other suitable joining method known in the art. The topsheet 40 is sanitary, clean in appearance, and somewhat opaque to hide bodily discharges collected in and absorbed by the absorbent core 44. The topsheet 40 further exhibits good strike-through and rewet characteristics permitting bodily discharges to rapidly penetrate through the topsheet 40 to the absorbent core 44, but not allow the body fluid to flow back through the topsheet 40 to the skin of the wearer. For example, some suitable materials that may be used for the topsheet 40 include nonwoven materials, perforated thermoplastic films, or combinations thereof. A nonwoven fabric made from polyester, polyethylene, polypropylene, bicomponent, nylon, rayon, or like fibers may be utilized. For instance, a white uniform spunbond material is particularly desirable because the color exhibits good masking properties to hide menses that has passed through it. U.S. Pat. No. 4,801,494 to Datta, et al. and U.S. Pat. No. 4,908,026 to Sukiennik, et al. teach various other cover materials that may be used in the present invention.

The topsheet 40 may also contain a plurality of apertures (not shown) formed therethrough to permit body fluid to pass more readily into the absorbent core 44. The apertures may be randomly or uniformly arranged throughout the topsheet 40, or they may be located only in the narrow longitudinal band or strip arranged along the longitudinal axis X--X of the absorbent article 20. The apertures permit rapid penetration of body fluid down into the absorbent core 44. The size, shape, diameter and number of apertures may be varied to suit one's particular needs.

As stated above, the absorbent article also includes a backsheet 42. The backsheet 42, which may also be formed in accordance with the present invention, is generally liquid-impermeable and designed to face the inner surface, i.e., the crotch portion of an undergarment (not shown). The backsheet 42 may permit a passage of air or vapor out of the absorbent article 20, while still blocking the passage of liquids. Any liquid-impermeable material may generally be utilized to form the backsheet 42. For example, one suitable material that may be utilized is a microembossed biodegradable film made in accordance with the present invention. In particular embodiments, a film is utilized that has a thickness in the range of about 0.2 mils to about 5.0 mils (about 5.08 to about 127 µm), and particularly between about 0.5 to about 3.0 mils (about 12.7 to about 76.2 µm).

The absorbent article 20 also contains an absorbent core 44 positioned between the topsheet 40 and the backsheet 42. The absorbent core 44 may be formed from a single absorbent member or from a composite containing separate and distinct absorbent members. It should be understood, however, that any number of absorbent members may be utilized in the present invention. For example, in one embodiment, the absorbent core 44 may contain an intake member (not shown) positioned between the topsheet 40 and a transfer delay member (not shown). The intake member may be made of a material that is capable of rapidly transferring, in the z-direction, body fluid that is delivered to the topsheet 40. The intake member may generally have any shape and/or size desired. In one embodiment, the intake member has a rectangular shape, with a length equal to or less than the overall length of the absorbent article 20, and a width less than the width of the absorbent article 20. For example, a length of between about 150 mm to about 300 mm and a width of between about 10 mm to about 60 mm may be utilized.

Any of a variety of different materials may be used for the intake member to accomplish the above-mentioned functions. The material may be synthetic, cellulosic, or a combination of synthetic and cellulosic materials. For example, airlaid cellulosic tissues may be suitable for use in the intake member. The airlaid cellulosic tissue may have a basis weight ranging from about 10 grams per square meter (gsm) to about 300 gsm, and in some embodiments, between about 100 gsm to about 250 gsm. In one embodiment, the airlaid cellulosic tissue has a basis weight of about 200 gsm. The airlaid tissue may be formed from hardwood and/or softwood fibers. The airlaid tissue has a fine pore structure and provides an excellent wicking capacity, especially for menses.

If desired, a transfer delay member (not shown) may be positioned vertically below the intake member. The transfer delay member may contain a material that is less hydrophilic than the other absorbent members, and may generally be characterized as being substantially hydrophobic. For example, the transfer delay member may be a nonwoven fibrous web composed of a relatively hydrophobic material, such as polypropylene, polyethylene, polyester or the like, and also may be composed of a blend of such materials. One example of a material suitable for the transfer delay member is a spunbond web composed of polypropylene, multilobal fibers. Further examples of suitable transfer delay member materials include spunbond webs composed of polypropylene fibers, which may be round, tri-lobal or poly-lobal in cross-sectional shape and which may be hollow or solid in structure. Typically the webs are bonded, such as by thermal bonding, over about 3% to about 30% of the web area. Other examples of suitable materials that may be used for the transfer delay member are described in U.S. Pat. No. 4,798,603 to Meyer, et al. and U.S. Pat. No. 5,248,309 to Serbiak, et al.. To adjust the performance of the invention, the transfer delay member may also be treated with a selected amount of surfactant to increase its initial wettability.

The transfer delay member may generally have any size, such as a length of about 150 mm to about 300 mm. Typically, the length of the transfer delay member is approximately equal to the length of the absorbent article 20. The transfer delay member may also be equal in width to the intake member, but is typically wider. For example, the width of the transfer delay member may be from between about 50 mm to about 75 mm, and particularly about 48 mm. The transfer delay member typically has a basis weight less than that of the other absorbent members. For example, the basis weight of the transfer delay member is typically less than about 150 grams per square meter (gsm), and in some embodiments, between about 10 gsm to about 100 gsm. In one particular embodiment, the transfer delay member is formed from a spunbonded web having a basis weight of about 30 gsm.

Besides the above-mentioned members, the absorbent core 44 may also include a composite absorbent member (not shown), such as a coform material. In this instance, fluids may be wicked from the transfer delay member into the composite absorbent member. The composite absorbent member may be formed separately from the intake member and/or transfer delay member, or may be formed simultaneously therewith. In one embodiment, for example, the composite absorbent member may be formed on the transfer delay member or intake member, which acts a carrier during the coform process described above.

Regardless of its particular construction, the absorbent article 20 typically contains an adhesive for securing to an undergarment. An adhesive may be provided at any location of the absorbent article 20, such as on the lower surface of the backsheet 42. In this particular embodiment, the backsheet 42 carries a longitudinally central strip of garment adhesive 54 covered before use by a peelable release liner 58, which may be a biodegradable film formed in accordance with the present invention. Each of the flaps 24 may also contain an adhesive 56 positioned adjacent to the distal edge 34 of the flap 24. A peelable release liner 57, which may also be formed in accordance with the present invention, may cover the adhesive 56 before use. Thus, when a user of the sanitary absorbent article 20 wishes to expose the adhesives 54 and 56 and secure the absorbent article 20 to the underside of an undergarment, the user simply peels away the liners 57 and 58 and disposed them in a disposal system.

Although various configurations of a release liner have been described above, it should be understood that other release liner configurations are also included within the scope of the present invention. Further, the present invention is by no means limited to release liners and the biodegradable film may be incorporated into a variety of different components of an absorbent article. For example, referring again to Fig. 3, the backsheet 42 of the napkin 20 may include the biodegradable film of the present invention. In such embodiments, the film may be used alone to form the backsheet 42 or laminated to one or more additional materials, such as a nonwoven web. The biodegradable film of the present invention may also be used in applications other than absorbent articles. For example, the film may be employed as an individual wrap, packaging pouch, or bag for the disposal of a variety of articles, such as food products, absorbent articles, etc. Various suitable pouch, wrap, or bag configurations for absorbent articles are disclosed, for instance, in U.S. Patent Nos. 6,716,203 to Sorebo, et al. and 6,380,445 to Moder, et al., as well as U.S. Patent Application Publication No. 2003/0116462 to Sorebo, et al..

The present invention may be better understood with reference to the following examples.

### Test Methods

### Tensile Properties:

The strip tensile strength values were determined in substantial accordance with ASTM Standard D-5034. A constant-rate-of-extension type of tensile tester was employed. The tensile testing system was a Sintech 1/D tensile tester, which is available from Sintech Corp. of Cary, North Carolina. The tensile tester was equipped with TESTWORKS 4.08B software from MTS Corporation to support the testing. An appropriate load cell was selected so that the tested value fell within the range of 10-90% of the full scale load. The film samples were initially cut into dog-bone shapes with a center width of 3.0 mm before testing. The samples were held between grips having a front and back face measuring 25.4 millimeters x 76 millimeters. The grip faces were rubberized, and the longer dimension of the grip was perpendicular to the direction of pull. The grip pressure was pneumatically maintained at a pressure of 40 pounds per square inch (275.8 kPa). The tensile test was run using a gauge length of 18.0 millimeters and a break sensitivity of 40%. Five samples were tested by applying the test load along the machine-direction and five samples were tested by applying the test load along the cross direction. During the test, samples were stretched at a crosshead speed of about 127 millimeters per minute until breakage occurred. The modulus, peak stress, peak strain (i.e., % strain at peak load), and elongation were measured and recorded.

### Scanning Electron Microscopy:

A method of plasma etching was used to prepare samples for structural profiles by SEM. Similar to wet etching, this method develops topography via differential etch rates of materials. Samples placed on an aluminum disc placed on ice were sectioned at room temperature by driving a fresh single edge razor through the thickness of the films with a mallet. Sections were cut either in the Extrusion Direction (ED) (i.e. machine direction, MD) or perpendicular to the extrusion direction (i.e. cross direction, CD). These preparations were inspected for typical and unusual features and micrographs illustrative of these observations were digitally captured.

Preliminary calculations predicted that a discernable contrast should be found between Ecoflex and oxidized starch. Sections of films without additional treatment were mounted to an aluminum stub using conductive carbon paint. Once dried, the mounted-film, save the section surface, was re-painted to provide two conductive paths to ground. The mounted sections were imaged using either a microchannel plate in high vacuum or a solid state detector in a partial vacuum for collection of backscattered electrons (native Backscattered Electron Imaging (BEI). Imaging conditions were optimized for contrast and resolution, representative images were digitally captured, and images selected.

Further samples of films were stained with osmium tetroxide in bell jar with 0.5-g of osmium tetroxide for forty-eight hours with a ventilation period of 4-days. Stained and ventilated films were sectioned and imaged as described above (OS BEI).

### Decorated Secondary Electron Imaging (SEI)

Sectioned films were oxygen plasma-decorated using an Emitech K1050X barrel reactor (available from Energy Beam Sciences, Inc., East Granby, Connecticut) that was operated at 40-W with an oxygen flow of 50-ml/minute, which was sufficient to produce the blue-white plasma indicative of an oxygen-rich plasma. Decorated sections were mounted to an aluminum stub with double-sided copper tape, sputter-coated with gold, and imaged using an Everhart-Thornley detector operated in secondary electron collection mode. Imaging conditions were optimized for contrast and resolution, and representative images were digitally captured.

### Materials

Cargill Gum™ 03460 is native (unoxidized) corn starch. Superfilm® 235D is oxidized corn starch. Both starches were purchased from Cargill (Minneapolis, MN).

Native (unoxidized) wheat starch, Midsol 50, and modified (oxidized) wheat starch, Pregel Adhere 2000, were purchased from MGP Ingredients, Inc. (Atchison, KS).

Oxidized starches were done using sodium hypochlorite in a suspension where the pH was maintained at an alkaline region.

ECONEER resin, EBP 203 (EBP), was purchased from ECONEER Co., Ltd. (Costa Mesa, CA). EBP 203 resin is a blend of Kondorax and Ecopel biodegradable polymer resins. Kondorax resin is composed of high molecular weight fibers, including but not limited to sugar cane, rice straw, barley straw, reed, corn stalk, coconut, pasture, and so forth. Ecopol is a thermal plastic aliphatic polyester copolymer that is biodegradable. The decomposition period for ECONEER resin ranges from 15 days at minimum to 12 months. ECONEER resin contains cheap and abundant renewable components.

Ecoflex® F BX 7011 resin was purchased from BASF (Mount Olive, NJ). Ecoflex® F BX 7011 resin is aliphatic-aromatic copolyester which is composed of three monomers: butanediol, adipic acid, and terephthalic acid. Ecoflex® F BX 7011 resin is biodegradable and commercially available, however, it contains no renewable content. Ecoflex® F BX 7011 was used to enhance film overall functionality.

Polybutylene succinate (PBS), GS-Pla AD92W, was purchased from the Mitsubishi Chemical Corporation (Tokyo, Japan).

Processing aids such as glycerin was purchased from Cognis Corporation (Cincinnati, OH). Mono-di-glyceride surfactant (Excel P-40S) was purchased from Kao Corporation (Tokyo, Japan). All these were used as processing aids.

### Thermoplastic Starch Preparation

### Example 1 (Reference)

Pregel Adhere 2000 oxidized wheat starch was converted into thermoplastic oxidized wheat starch using glycerin as a plasticizer and Excel P-40S as a surfactant according to the percentages provided in Table 1. A Thermo Prism™ USLAB 16 twin screw extruder (Thermo Electron Corporation, Stone, England) was used to complete the processing. The extruder has eleven zones: zone 0 is a feeding zone where the materials from a K-Tron feeder (K-Tron North America, Pitman, NJ) were accepted and conveyed to the zone 1, 2, etc. till zone 9. These zones are kneading sections of the twin screws, and zone 10 is a die located at the end of the extruder. The temperature setup for Example 1 was 90, 100, 115, 125, 130, 130, 130, 125, and 120 °C from zones 1 to 9. The die temperature was 115 °C. The screw rotational speed was 150 rpm. The oxidized wheat starch, after mixing with Excel P-40S, was fed at 1.5 lb/hr (0.675 kg/hr). Glycerin was pumped into zone 1 using a gear pump (Bodine Electric Company, Grand Island, NY). At these conditions, the torque ranged from 82 to 86%, and the pressure was 10~11 bars (1-1.1 MPa). All these processing conditions were summarized as Example 1 in Table 1. When the strand was formed, it was cooled down through a convey belt (Bondie Electric Company, Chicago, IL). A pelletizer (Emerson Industrial Controls, Grand Island, NY) was used to cut the strand to produce thermoplastic oxidized wheat starch pellets, which were then collected and sealed in a plastic bag.

### Example 2 (Comparative)

Native wheat starch, Midsol 50, was processed similarly using the same equipment shown in Example 1. The processing parameters are shown in Table 1.

### Example 3 (Reference)

Oxidized corn starch, Superfilm® 235D, was converted into thermoplastic oxidized starch using the same equipment shown in Example 1. The processing parameters are shown in Table 1.

### Example 4 (Comparative)

Native corn starch was converted into thermoplastic starch using the same equipment shown in Example 2. The processing parameters are shown in Table 1.

### Film Preparation

Ecoflex® F BX 7011 resin control film was cast for Example 5 (control) using for Thermo Prism™ USLAB 16 twin screw extruder (Thermo Electron Corporation, Stone, England), after attaching a 4" inch (10.16 cm) film die. The temperature profile for the film casting was shown in Table 2, including other processing conditions such melt pressure, torque, and screw rotational speed. The control film thickness was about 1.5 and 2 mils (38.1 and 50.8 µm), respectively. Examples 6 to 19 are film blends using various biodegradable resins and thermoplastic starch blends, the compositions of which and processing conditions for which are specified in Tables 2 and 3. Examples 6-9 and 12-19 are comparative, Examples 10 and 11 are examples of the invention.

Examples 5 - 10 processed well. Thin films were successfully produced from thermoplastic oxidized starch and copolyester blends containing 20 wt.%, 30 wt.%, 40 wt.%, and 45 wt.% of thermoplastic oxidized starch. Surprisingly, thin film were also successfully produced from blends containing a majority of thermoplastic oxidized starch (Example 10) at 60% by weight.

For Example 11, films more than 4~5 mils (101.6~127µm) or greater were able to be melt cast, though the mechanical integrity of Example 11 films was not as good as those films produced from Examples 6 to 10. Therefore, no tensile testing was performed on the Example 11 films.

Examples 12 and 13 used a blend of native corn thermoplastic starch and Ecoflex® F BX 7011 resin for film casting. When the ratio of native corn thermoplastic starch was greater than 50 or 60% in the blend, the flexible film could not be formed. In comparison to Example 10, Examples 12 and 13 demonstrate that oxidation of the wheat starch allows for providing a film-forming formulation that includes a majority of thermoplastic oxidized starch in the blends.

For Examples 14 and 15, it was very difficult to form films when the composition included thermoplastic native wheat starch greater than 35 wt. %.

Examples 16 to 17 used oxidized corn starch from Cargill, Inc. to cast films. The film can be formed only when Ecoflex® F BX 7011 resin was a majority component. When oxidized corn starch was greater than 50%, the thin and flexible films could not be formed.

Example 18 is a blend of 55% ECONEER EBP 203 and 45% thermoplastic oxidized starch from Example 1. The film produced had thickness greater than 3 mils (76.2 µm).

Example 19 is the blend of 60% PBS and 40% thermoplastic oxidized starch from Example 1. The thin film was obtained through melt casting, and they were much better than those from Example 18.

### Film Mechanical Properties

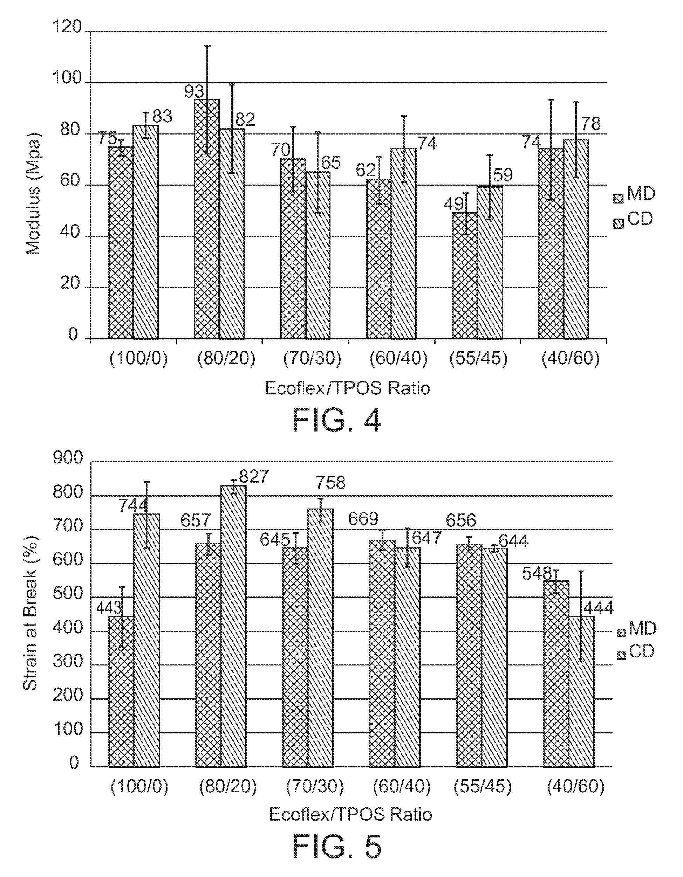

Table 4 listed all thermoplastic film tensile properties from Examples 5 to 19 except for Example 11, which was unable to be tested. The film modulus and elongation data for Examples 5 to 10 are plotted and shown in Figs. 4 and 5, respectively. The error bar represents one standard deviation. The film modulus appeared to be stable without deterioration of film stiffness as the amount of thermoplastic oxidized starch increases shown in Fig. 4, indicating the blends are completely miscible. On the other hand, the film elongation basically held relatively high elongation in all films as amount of thermoplastic oxidized starch increased. The films incorporating thermoplastic oxidized starch had overall better elongation, with the exception of the 40/60 blend, which is still not too much different from pure Ecoflex® F BX 7011 resin films.

Examples 12 and 13 used native corn thermoplastic starch, where film modulus is higher than those of the films from Examples 5 to 10, regardless amount of Ecoflex® F BX 7011 resin in the blend, indicating the films from Examples 12 and 13 are less flexible. Peak stress and elongation were also reduced particularly for the films in Example 13.

Examples 14 and 15 used native wheat thermoplastic starch, with resulting film modulus at a similar level to those of Examples 5 to 10, but the amount of starch in the blends is relatively low.

Examples 16 and 17 used oxidized corn starch. Correspondingly, they should be compared to Examples 9 and 8, respectively, where Ecoflex® F BX 7011 resin and TPOS ratios are the same. The oxidized corn starch did not perform as well as the oxidized wheat starch in terms of the film modulus and thin film processability.

Example 18 is the blend of ECONEER EBP 203 and oxidized wheat starch (55/45), which resulted in a thick film. The film tensile strength was less as well.

Example 19 is the blend of PBS and oxidized wheat starch (60/40). The film tensile values are better than those in Example 18, but poorer than the blends of Ecoflex® F BX 7011 resin and oxidized wheat starch demonstrated by Examples 6 to 10.

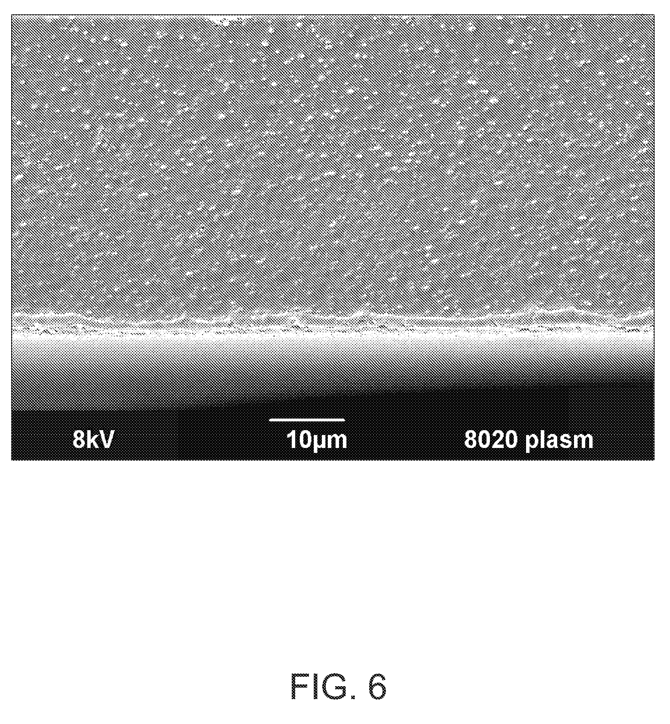

Fig. 6 depicts an SEM photo of an Example 6 film (20% thermoplastic oxidized starch), where Ecoflex® F BX 7011 resin forms a continuous phase, and thermoplastic oxidized starch is evenly distributed across the film.

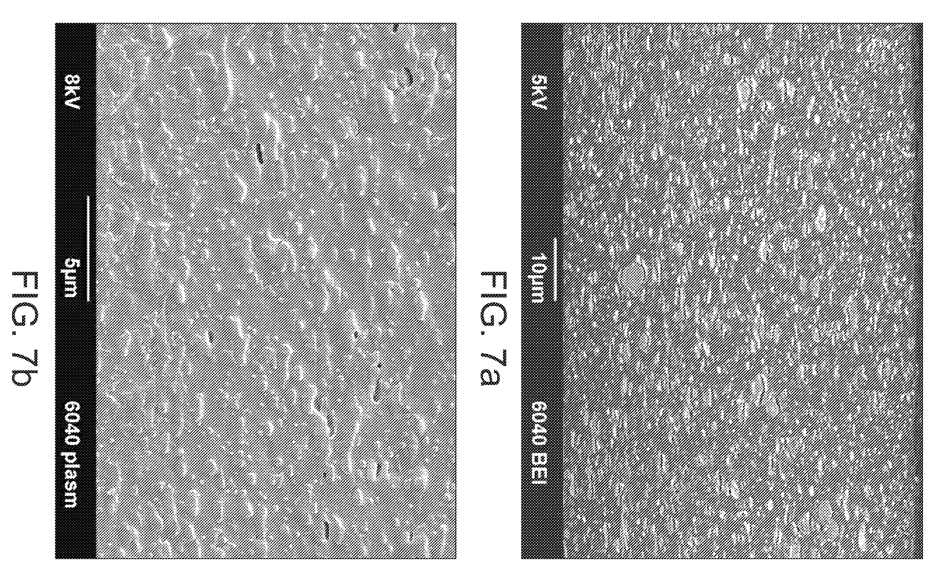

Fig. 7a depicts a back scattered electron image (BEI) of an Example 8 film (40% thermoplastic oxidized starch), where Ecoflex® F BX 7011 resin still serves as the continuous (matrix) phase and the starch serves as the dispersed phase. The dark area indicates Ecoflex® F BX 7011 resin and the bright areas represent thermoplastic oxidized starch, noting that this SEM was obtained using the osmium-stained BEI technique.

Fig. 7b depicts a secondary electron image of an Example 8 film. The film was etched for 4 minute by plasma. During the plasma treatment, the Ecoflex® F BX 7011 resin was etched at a higher rate than thermoplastic oxidized starch phase. The presence of the etched voids confirmed that the continuous phase was the Ecoflex® F BX 7011 resin phase, while the dispersed phase was the thermoplastic oxidized starch phase. On this cross direction (CD) image, the size of the dispersed phase ranged quite widely, from sub-micron size to several microns. The nearly circular cross sections of some dispersed thermoplastic oxidized starch structure in Fig. 7b suggested that some of the dispersed thermoplastic oxidized starch phase could have a nearly circular fiber-like structure, although there were also some thermoplastic oxidized starch moieties that appear as ribbon-like structures in the cross direction.

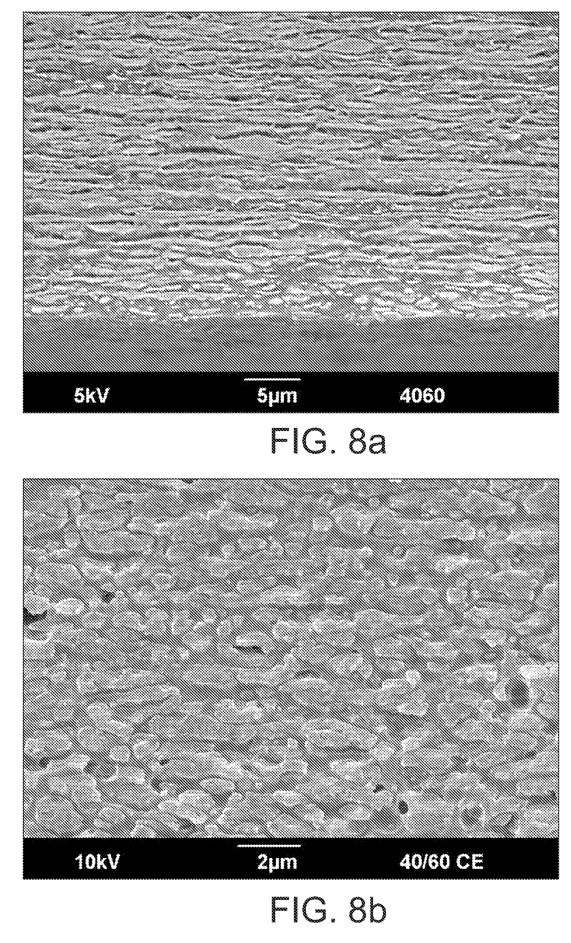

Fig. 8a depicts a back scattered electron image (BEI) of an Example 10 film (60% thermoplastic oxidized starch, 40% Ecoflex® F BX 7011 resin). The majority material in the blend is thermoplastic oxidized starch at 60%. The thermoplastic oxidized starch phases were interconnected as highly extended lamellar shaped dispersed structures. The film has a microstructural feature which mimics microlayered film laminate in which finely divided layers of copolyester with a fine thickness of submicron is inter-laminated between microlayers of thermoplastic oxidized starch in thickness ranging from submicron to several microns. It is believed that this type of unique microstructure led the film to have good mechanical properties because Ecoflex® F BX 7011 resin still serves as a continuous matrix phase as shown in Fig. 8a even though the thermoplastic oxidized starch is predominating in the blend. This SEM was also obtained using osmium-stained BEI technique.

Fig. 8b depicts a secondary electron image of a cross-directional section of an Example 10 film. The film was etched for 4 minute by plasma. During the plasma treatment, the Ecoflex® F BX 7011 resin was etched at a higher rate than thermoplastic oxidized starch phase. The presence of the etched voids confirmed that the continuous phase was the Ecoflex® F BX 7011 resin phase, while the dispersed phase was the thermoplastic oxidized starch phase. It is evident that the dispersed phase makes up the majority of the cross section. The elongated cross sections of many of the dispersed thermoplastic oxidized starch structures in Fig. 8b suggest that some of the dispersed thermoplastic oxidized starch phase could have a ribbon-like structure, although there were also some thermoplastic oxidized starch moieties that appear as ribbon-like structures in the cross direction.

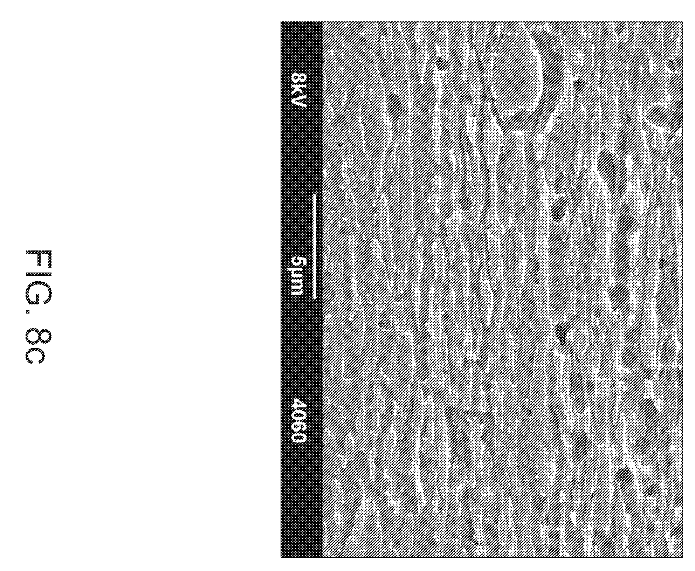

Fig. 8c depicts a secondary electron image of a machine direction section of an Example 10 film. The film was etched for 4 minute by plasma. During the plasma treatment, the Ecoflex® F BX 7011 resin was etched at a higher rate than thermoplastic oxidized starch phase. The presence of the etched voids confirmed that the continuous phase was the Ecoflex® F BX 7011 resin phase, while the dispersed phase was the thermoplastic oxidized starch phase. It is evident that the dispersed phase makes up the majority of the cross section. The elongated cross sections of many of the dispersed thermoplastic oxidized starch structures in Fig. 8c suggest that some of the dispersed thermoplastic oxidized starch phase could have a lamellar structure, with the some lamellar structures having a length of greater than 5 microns and even some greater than 10 microns. In between the thermoplastic oxidized starch structures, the Ecoflex® F BX 7011 resin was thinned considerably, contributing to the overall ductility of the film.

The starch molecular weight distributions are shown in Table 5. The polydispersity index (Mw/Mn) for oxidized wheat or corn starch is higher than those of native wheat or corn starch. The higher polydispersity index indicates that the molecular weight distribution for the oxidized starch is much broader relatively to native starch. Without being bound by theory, it may be that an increase in polydispersity index improves a starch's capability to form a thin and flexible film.

While the invention has been described in detail with respect to the specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims.

**Table 1. Thermoplastic Starch Processing Conditions**

| | Mixture | Blend Composition | | | Extruder | | | | Extruder Temperature Profile (°C) | | | | | | | | |
|---|---|---|---|---|---|---|---|---|---|---|---|---|---|---|---|---|---|
| Sample No. | Feeding Rate | Starch | Excel P-40S | Glycerin | Speed | | | | | | | | | | | Pₘₑₗₜ | Torque |
| | (lb/hr) | % | % | % | (rpm) | T₁ | T₂ | T₃ | T₄ | T₅ | T₆ | T₇ | T₈ | T₉ | T_{die} | (bar) | (%) |
| Example 1 | 1.5 | 78.4 | 1.6 | 20 | 150 | 90 | 100 | 115 | 125 | 130 | 130 | 130 | 125 | 120 | 115 | 10~11 | 82~86 |
| Example 2 | 1.8 | 73.5 | 1.5 | 25 | 150 | 90 | 100 | 110 | 125 | 130 | 130 | 120 | 125 | 120 | 115 | 35~37 | 55~58 |
| Example 3 | 1,9 | 74,5 | 1.5 | 24 | 150 | 95 | 110 | 125 | 135 | 150 | 150 | 150 | 135 | 125 | 115 | 30~35 | 65~70 |
| Example 4 | 23 | 76,4 | 1,6 | 22 | 150 | 90 | 100 | 115 | 120 | 130 | 130 | 130 | 120 | 115 | 110 | 9~10 | 55~60 |

| | | | | | | | | | | | | | | | | | |
|---|---|---|---|---|---|---|---|---|---|---|---|---|---|---|---|---|---|
| (1 lb/hr = 0.45 kg/hr, 1 bar = 100 kPa) | | | | | | | | | | | | | | | | | |

**Table 2. Wheat -Derived Thermoplastic Oxidized Starch and Co-Polyester Blend Film Processing Conditions**

| | Mixture | Blend Composition | | Extruder | | | | Extruder Temperature Profile (°C) | | | | | | | | |
|---|---|---|---|---|---|---|---|---|---|---|---|---|---|---|---|---|
| Sample No. | Feeding Rate | Pregel Adhere 2000 TPS | Ecoflex | Speed | | | | | | | | | | | Pₘₑₜ | Torque |
| | (lb/hr) | % | % | (rpm) | T₁ | T₂ | T₃ | T₄ | T₅ | T₆ | T₇ | T₈ | T₉ | T_{die} | (bar) | (%) |
| Example 5 | | 0 | 100 | 150 | 120 | 130 | 140 | 155 | 155 | 160 | 155 | 155 | 155 | 150 | 14~15 | 70~75 |
| Example | | 20 | 80 | 150 | 120 | 130 | 140 | 145 | 150 | 150 | 150 | 150 | 145 | 140 | 6~7 | 55~58 |
| Example 7 | | 30 | 70 | 150 | 120 | 130 | 140 | 145 | 150 | 150 | 150 | 150 | 145 | 140 | 7~8 | 58~63 |
| Example 8 | 2.5 | 40 | 60 | 150 | 120 | 130 | 140 | 145 | 150 | 150 | 150 | 150 | 145 | 140 | 9~10 | 60~63 |
| Exampe 9 | | 45 | 55 | 150 | 120 | 130 | 140 | 145 | 150 | 150 | 150 | 150 | 145 | 140 | 6~7 | 50~52 |
| Example 10 | | 60 | 40 | 150 | 120 | 130 | 140 | 145 | 150 | 150 | 150 | 150 | 145 | 140 | 10~11 | 50~55 |
| Example 11 | | 70 | 30 | 150 | 120 | 130 | 140 | 145 | 150 | 150 | 150 | 150 | 145 | 140 | 9~10 | 53~56 |

| | | | | | | | | | | | | | | | | |
|---|---|---|---|---|---|---|---|---|---|---|---|---|---|---|---|---|
| Examples 5-9 = comparative Examples 10-11 = invention (1 lb/hr = 0.45 kg/hr, 1 bar = 100 kPa) | | | | | | | | | | | | | | | | |

**Table 3. Starch-Based Thermoplastic Co-polyester Film Processing Conditions**

| | Mixture | Blend Composition | | Extruder | | | | Extruder Temperature Profile (°C) | | | | | | | | |
|---|---|---|---|---|---|---|---|---|---|---|---|---|---|---|---|---|
| Sample No. | Feeding Rate | TPS | Ecoflex | Speed | | | | | | | | | | | Pₘₑₜ | Torque |
| | (lb/hr) | | % | (rpm) | T₁ | T₂ | T₃ | T₄ | T₅ | T₆ | T₇ | T₈ | T₉ | T_{die} | (bar) | (%) |
| Example 12 | 2.5 | 30% Example 4 | 70 | 150 | 120 | 130 | 140 | 150 | 160 | 160 | 160 | 155 | 150 | 145 | 9~10 | 60~64 |
| Example 13 | | 45% Example 4 | 55 | 150 | 120 | 130 | 140 | 150 | 160 | 160 | 160 | 155 | 150 | 145 | 10~11 | 65~70 |
| Example 14 | 2.5 | 20% Example 2 | 80 | 150 | 120 | 130 | 140 | 150 | 160 | 160 | 160 | 155 | 150 | 145 | 7~8 | 55~58 |
| Example 15 | | 30% Example 2 | 70 | 150 | 120 | 130 | 140 | 145 | 150 | 150 | 150 | 150 | 145 | 140 | 8~9 | 53~57 |
| Example 16 | 2.5 | 45% Example 3 | 55 | 150 | 120 | 130 | 140 | 145 | 150 | 150 | 150 | 150 | 145 | 135 | 6~7 | 48~50 |
| Example 17 | | 40% Example 3 | 60 | 150 | 120 | 130 | 140 | 145 | 150 | 160 | 160 | 155 | 150 | 145 | 7~6 | 55~58 |
| Example 18 | 2.5 | 45% Example 1 | 55% EBP | 150 | 120 | 130 | 140 | 145 | 150 | 150 | 150 | 150 | 145 | 140 | 9~10 | 50~54 |
| Example 19 | 2.5 | 40% Example 1 | 60% PBS | 150 | *120* | 130 | 140 | 150 | 160 | 160 | 160 | 155 | 150 | 145 | 11~12 | 53~55 |

| | | | | | | | | | | | | | | | | |
|---|---|---|---|---|---|---|---|---|---|---|---|---|---|---|---|---|
| Examples 12-19 = comparative (1 lb/hr = 0.45 kg/hr, 1 bar = 100kPa) | | | | | | | | | | | | | | | | |

**Table 4. Mechanical Properties of Thermoplastic Oxidized Starch-Aliphatic Aromatic Copolyester Blend Films**

| Sample No. | Sample Description | Composition | Film Mechanical Properties | | | | | | | |
|---|---|---|---|---|---|---|---|---|---|---|
| | | | Film Thickness | | Modulus (MPa) | | Peak Stress (MPa) | | Elongation (%) | |
| | | | MD (mil) | CD (mil) | MD | CD | MD | CD | MD | CD |
| Example 5 | Ecoflex F BX 7011 | 100/0 | 1.3 | 1.3 | 74.6 | 83.5 | 42.1 | 35.3 | 442.7 | 744.2 |
| Example 6 | Ecoflex/Example 1 | 80/20 | 1.3 | 1.2 | 93.4 | 82.2 | 34.4 | 30.2 | 657.3 | 827.4 |
| Example 7 | Ecoflex/Examole 1 | 70/30 | 1.1 | 1.2 | 70.1 | 65.0 | 30.1 | 21.7 | 645.2 | 758.4 |
| Example 8 | Ecoflex/Example 1 | 60/40 | 1.4 | 1.2 | 61.9 | 74.3 | 29.8 | 17.4 | 669.2 | 646.8 |
| Example 9 | Ecoflex/Example 1 | 55/45 | 1.4 | 1.4 | 49.0 | 59.3 | 24.0 | 13.9 | 656.3 | 644.4 |
| Example 10 | Ecoflex/Example 1 | 40/60 | 1.4 | 1.7 | 73.9 | 77.7 | 17.3 | 8.1 | 547.5 | 444.5 |
| Example 12 | Eoflex/Example 4 | 70/30 | 1.8 | 1.7 | 106.3 | 112.3 | 25.7 | 15.3 | 690.8 | 597,9 |
| Example 13 | | 55/45 | 1.4 | 1.3 | 255.9 | 255.9 | 16,0 | 8.0 | 435.4 | 36.8 |
| Example 14 | Ecoflex/Example 2 | 80/20 | 3.0 | 2.6 | 76.8 | 79.0 | 24.0 | 15.1 | 782.5 | 556.8 |
| Example 15 | | 70/30 | 3.0 | 2.6 | 70.7 | 107.2 | 21.9 | 11.6 | 695.1 | 433.7 |
| Example 16 | Ecoflex/Example 3 | 55/45 | 1.6 | 1.2 | 72.1 | 103.2 | 25.4 | 11.3 | 548.9 | 385.0 |
| Example 17 | | 60/40 | 1.6 | 1.5 | 118.3 | 96.1 | 29.2 | 16.4 | 601.8 | 600.7 |
| Example 18 | EBP/Example 1 | 55/45 | 3.3 | 3.2 | 144.6 | 93.1 | 9.6 | 4.7 | 164.4 | 10.4 |
| Example 19 | PBS/Example 1 | 60/40 | 1.5 | 1.5 | 110.1 | 129.6 | 22.8 | 12.6 | 375.3 | 394.2 |

| | | | | | | | | | | |
|---|---|---|---|---|---|---|---|---|---|---|
| Exaples 5-9 and 12-19 = comparative Example 10 = invention (1 mil = 25.4 µm) | | | | | | | | | | |

**Table 5. Starch Molecular Weight Distribution for Starch Sample**

| Starch Sample | Mn | M_{w} | M_{z} | M_{w}/Mₙ |
|---|---|---|---|---|
| Midsol 50 | 183,500 | 7,731,000 | 21,770,000 | 42 |
| Pregel Adhere 2000 | 28,300 | 3,224,000 | 10,670,000 | 114 |
| Cargill Gum ™03460 | 182,600 | 3,231,000 | 12,41,000 | 18 |
| Superfilm 235®D | 35,500 | 2,455,000 | 9,165,000 | 69 |

## Claims

1. A biodegradable film, the film comprising:
from 1 wt.% to 49 wt. % by weight of the film of at least one biodegradable polyester; and
from 46 wt.% to 75 wt.% by weight of the film of a thermoplastic oxidized starch comprising at least one oxidized corn or wheat starch and at least one plasticizer;
wherein the wt.% by weight of the film of the biodegradable polyester is less than the wt.% by weight of the film of the thermoplastic oxidized starch;
wherein the biodegradability of the film and the polyester are determined according to ASTM Test Method 5338.92.

2. The biodegradable film of claim 1, wherein the biodegradable polyester is an aliphatic polyester, aromatic polyester or aliphatic-aromatic copolyester.

3. The biodegradable film of claim 1, wherein the biodegradable polyester constitutes from 20 wt.% to 43 wt.% of the film.

4. The biodegradable film of claim 1, wherein the oxidized corn or wheat starch constitutes from 45 wt.% to 75 wt.% of the film.

5. The biodegradable film of claim 1, wherein the plasticizer constitutes from 5 wt.% to 30 wt.% of the film.

6. The biodegradable film of claim 1, wherein the oxidized corn or wheat starch is an oxidized modified corn or wheat starch.

7. The biodegradable film of claim 6, wherein the oxidized modified corn or wheat starch is an oxidized corn or wheat starch ester, an oxidized corn or wheat starch ether, or an oxidized hydrolyzed corn or wheat starch.

8. The biodegradable film of claim 1, wherein the plasticizer is a polyhydric alcohol.

9. The biodegradable film of claim 1, wherein the film has a thickness of 50 micrometers or less.

10. A release liner comprising the biodegradable film of claim 1 and a release agent coated onto a surface thereof.

11. An absorbent article comprising the biodegradable film of claim 1.

12. The absorbent article of claim 11 which is a diaper, training pants, absorbent underpants, incontinence article, feminine hygiene product, swim wear, baby wipe, medical absorbent garment, fenestration material, underpad, bedpad, bandage, absorbent drape, medical wipe or food service wiper.

13. The absorbent article of claim 11, comprising a body portion that includes a liquid permeable topsheet, a generally liquid impermeable backsheet and an absorbent positioned between the backsheet and the topsheet, wherein the backsheet includes the biodegradable film.

14. A pouch, wrap, or bag comprising the biodegradable film of claim 1.

## Patentansprüche

1. Biologisch abbaubarer Film, wobei der Film umfasst:
1 Gew.-% bis 49 Gew.-%, bezogen auf das Gewicht des Films, zumindest eines biologisch abbaubaren Polyesters; und
46 Gew.-% bis 75 Gew.-%, bezogen auf das Gewicht des Films, einer thermoplastischen, oxidierten Stärke, umfassend zumindest eine oxidierte Mais- oder Weizenstärke und zumindest einen Weichmacher;
wobei die Gew.-%, bezogen auf das Gewicht des Films, des biologisch abbaubaren Polyesters geringer sind als die Gew.-%, bezogen auf das Gewicht des Films, der thermoplastischen, oxidierten Stärke;
wobei die biologische Abbaubarkeit des Films und des Polyesters nach ASTM Testmethode 5338.92 bestimmt ist.

2. Biologisch abbaubarer Film nach Anspruch 1, wobei der biologisch abbaubare Polyester ein aliphatischer Polyester, aromatischer Polyester oder aliphatischaromatischer Polyester ist.

3. Biologisch abbaubarer Film nach Anspruch 1, wobei der biologisch abbaubare Polyester 20 Gew.-% bis 43 Gew.-% des Films bildet.

4. Biologisch abbaubarer Film nach Anspruch 1, wobei die oxidierte Mais- oder Weizenstärke 45 Gew.-% bis 75 Gew.-% des Films bildet.

5. Biologisch abbaubarer Film nach Anspruch 1, wobei der Weichmacher 5 Gew.-% bis 30 Gew.-% des Films bildet.

6. Biologisch abbaubarer Film nach Anspruch 1, wobei die oxidierte Mais- oder Weizenstärke eine oxidierte modifizierte Mais- oder Weizenstärke ist.

7. Biologisch abbaubarer Film nach Anspruch 6, wobei die oxidierte modifizierte Mais- oder Weizenstärke ein oxidierter modifizierter Mais- oder Weizenstärkeester, ein oxidierter modifizierter Mais- oder Weizenstärkeether oder eine oxidierte hydrolysierte Mais- oder Weizenstärke ist.

8. Biologisch abbaubarer Film nach Anspruch 1, wobei der Weichmacher ein mehrwertiger Alkohol ist.

9. Biologisch abbaubarer Film nach Anspruch 1, wobei der Film eine Dicke von 50 Mikrometer oder weniger hat.

10. Trennschicht, umfassend den biologisch abbaubaren Film nach Anspruch 1 und ein Trennmittel, das an dessen Oberfläche aufgetragen ist.

11. Saugfähiger Artikel, umfassend den biologisch abbaubaren Film nach Anspruch 1.

12. Saugfähiger Artikel nach Anspruch 11, der eine Windel, ein Trainingshöschen, eine saugfähige Unterhose, ein Inkontinenzartikel, ein Damenhygieneprodukt, eine Badebekleidung, ein Baby-Wischtuch, ein medizinisches saugfähiges Kleidungsstück, ein Fensterbaumaterial, eine Unterlage, eine Betteinlage, eine Bandage, ein saugfähiger Verband, ein medizinisches Wischtuch oder ein Wischtuch in der Gastronomie ist.

13. Saugfähiger Artikel nach Anspruch 11, umfassend einen Körperabschnitt, der eine flüssigkeitsdurchlässige Deckschicht, eine im Allgemeinen flüssigkeitsundurchlässige Unterlagenschicht und ein saugfähiges Material, das zwischen der Unterlagenschicht und der Deckschicht positioniert ist, enthält, wobei die Unterlagenschicht den biologisch abbaubaren Film enthält.

14. Beutel, Hülle oder Sack, umfassend den biologisch abbaubaren Film nach Anspruch 1.

## Revendications

1. Film biodégradable, le film comprenant :
1 % en poids à 49 % en poids du film d'au moins un polyester biodégradable ; et
46 % en poids à 75 % en poids du film d'un amidon thermoplastique oxydé comprenant au moins un amidon oxydé de maïs ou de blé et au moins un plastifiant ;
dans lequel le % en poids du film de polyester biodégradable est inférieur au % en poids du film d'amidon thermoplastique oxydé ;
dans lequel la biodégradabilité du film et du polyester sont déterminées selon la norme ASTM Procédé d'Essai 5338.92.

2. Film biodégradable selon la revendication 1, dans lequel le polyester biodégradable est un polyester aliphatique, un polyester aromatique ou un copolyester aliphatique-aromatique.

3. Film biodégradable selon la revendication 1, dans lequel le polyester biodégradable constitue 20 % en poids à 43 % en poids du film.

4. Film biodégradable selon la revendication 1, dans lequel l'amidon oxydé de maïs ou de blé constitue 45 % en poids à 75 % en poids du film.

5. Film biodégradable selon la revendication 1, dans lequel le plastifiant constitue 5 % en poids à 30 % en poids du film.

6. Film biodégradable selon la revendication 1, dans lequel l'amidon oxydé de maïs ou de blé est un amidon oxydé de maïs ou de blé modifié.

7. Film biodégradable selon la revendication 6, dans lequel l'amidon oxydé de maïs ou de blé modifié est un ester d'amidon oxydé de maïs ou de blé, un éther d'amidon oxydé de maïs ou de blé ou un amidon oxydé de maïs ou de blé hydrolysé.

8. Film biodégradable selon la revendication 1, dans lequel le plastifiant est un alcool polyvalent.

9. Film biodégradable selon la revendication 1, dans lequel le film a une épaisseur de 50 micromètres ou moins.

10. Garniture antiadhésive comprenant le film biodégradable selon la revendication 1 et un agent antiadhésif appliqué sur l'une de ses surfaces.

11. Article absorbant comprenant le film biodégradable de la revendication 1.

12. Article absorbant selon la revendication 11, qui est formé d'une couche-culotte, d'un caleçon, d'un slip absorbant, d'un article pour incontinence, d'un produit d'hygiène féminine, d'un maillot de bain, d'une couche pour bébé, d'un vêtement médical absorbant, d'un matériau de fenêtrage, d'une serviette hygiénique, d'une alèse, d'un pansement, d'une alèse absorbante, d'une couche médicale ou d'une serviette de service de restauration.

13. Article absorbant selon la revendication 11, comprenant une partie de corps qui comprend une couche supérieure perméable aux liquides, une couche inférieure généralement imperméable aux liquides et un absorbant positionné entre la couche inférieure et la couche supérieure, dans lequel la couche inférieure comprend le film biodégradable.

14. Sac, emballage ou sachet comprenant le film biodégradable de la revendication 1.
